# EUROPEAN PATENT SPECIFICATION

(11) **EP 2 373 819 B1**
(45) Date of publication and mention of the grant of the patent: **14.01.2015**
(21) Application number: 09799601.1
(22) Date of filing: 14.12.2009
(51) Int. Cl.: C12Q 1/70

(54) **MULTIPARAMETER ASSAY**
TESTVERFAHREN MIT MEHREREN GRÖSSEN
DOSAGE À PLUSIEURS PARAMÈTRES

(30) Priority: 15.12.2008 GB 0822817
(43) Date of publication of application: 12.10.2011
(73) Proprietor: PathoFinder Holding BV, 6229 EG Maastricht (NL)
(72) Inventor: REIJANS, Martin, 6229 EG Maastricht (NL); THEELEN, Wendy, 6200 MD Maastricht (NL); HOPMAN, Anton, 6200 MD Maastricht (NL)
(74) Representative: Laenen, Bart Roger Albert
(86) International application number: PCT/EP2009/067131
(87) International publication number: WO 2010/069939

(56) References cited:
- WO-A1-2006/038753
- YOSHIDA T ET AL: "Quantitative real-time polymerase chain reaction analysis of the type distribution, viral load, and physical status of human papillomavirus in liquid-based cytology samples from cervical lesions." INTERNATIONAL JOURNAL OF GYNECOLOGICAL CANCER : OFFICIAL JOURNAL OF THE INTERNATIONAL GYNECOLOGICAL CANCER SOCIETY 2008 JAN-FEB, vol. 18, no. 1, January 2008 (2008-01), pages 121-127, XP002574636 ISSN: 1525-1438
- ANDERSSON SONIA ET AL: "Expression of E6/E7 mRNA from 'high risk' human papillomavirus in relation to CIN grade, viral load and P16(INK4a)" INTERNATIONAL JOURNAL OF ONCOLOGY, vol. 29, no. 3, September 2006 (2006-09), pages 705-711, XP002574637 ISSN: 1019-6439
- WILTING S M ET AL: "Increased gene copy numbers at chromosome 20q are frequent in both squamous cell carcinomas and adenocarcinomas of the cervix" JOURNAL OF PATHOLOGY, vol. 209, no. 2, June 2006 (2006-06), pages 220-230, XP002574638 ISSN: 0022-3417
- LUKASZUK KRZYSZTOF ET AL: "Human papillomavirus type 16 status in cervical carcinoma cell DNA assayed by multiplex PCR." JOURNAL OF CLINICAL MICROBIOLOGY FEB 2003, vol. 41, no. 2, February 2003 (2003-02), pages 608-612, XP002574639 ISSN: 0095-1137
- HOPMAN A H N ET AL: "Genomic integration of oncogenic HPV and gain of the human telomerase gene TERC at 3q26 are strongly associated events in the progression of uterine cervical dysplasia to invasive cancer" JOURNAL OF PATHOLOGY, vol. 210, no. 4, December 2006 (2006-12), pages 412-419, XP002574657 ISSN: 0022-3417
- THEELEN W ET AL: "A new multiparameter assay to assess HPV 16/18, viral load and physical status together with gain of telomerase genes in HPV-related cancers" INTERNATIONAL JOURNAL OF CANCER 20100215 WILEY-LISS INC. USA, vol. 126, no. 4, 15 February 2010 (2010-02-15), pages 959-975, XP002574640

### SEQUENCE LISTING

<110> Pathofinder BV
<120> MULTIPARAMETER ASSAY
<130> PATH-001
<150> GB0822817.3
   <151> 2008-12-15
<160> 45
<170> PatentIn version 3.3
<210> 1
   <211> 61
   <212> DNA
   <213> Artificial
<220>
   <223> MLPA Primer/Probe
<400> 1
   gggcaccgaa gaaacacaga cgactatcca gcgaccaaga tcagagccag acaccggaaa 60
   c 61
<210> 2
   <211> 73
   <212> DNA
   <213> Artificial
<220>
   <223> MLPA Primer
<400> 2
   gccaacgttt aaatgtgtgt caggacaaaa tactaacaca ttatgaaaat gatagtacag 60
   acctacgtga cca 73
<210> 3
   <211> 76
   <212> DNA
   <213> Artificial
<220>
   <223> MLPA Primer
<400> 3
   taatattaga atgtgtgtac tgcaagcaac agttactgcg acgtgaggta tatgactttg 60
   cttttcggga tttatg 76
<210> 4
   <211> 64
   <212> DNA
   <213> Artificial
<220>
   <223> MLPA Primer
<400> 4
   ctggacaagc agaaccggac agagcccatt acaatattgt aaccttttgt tgcaagtgtg 60
   actc 64
<210> 5
   <211> 67
   <212> DNA
   <213> Artificial
<220>
   <223> MLPA Primer
<400> 5
   gagaagcagc attgtggacc tgtcaaccca cttctcggtg cagctacacc tacaggcaac 60
   aacaaaa 67
<210> 6
   <211> 70
   <212> DNA
   <213> Artificial
<220>
   <223> MLPA Primer
<400> 6
   ggacagtgtg tattatatga ctgatgcagg aacatgggac aaaacggcta cctgtgtaag 60
   tcacagggga 70
<210> 7
   <211> 58
   <212> DNA
   <213> Artificial
<220>
   <223> MLPA Primer
<400> 7
   gcgctttgag gatccaacac ggcgacccta caagctacct gatctgtgca cggaactg 58
<210> 8
   <211> 76
   <212> DNA
   <213> Artificial
<220>
   <223> MLPA Primer
<400> 8
   gcaattaagc gactcagagg aagaaaacga tgaaatagat ggagttaatc atcaacattt 60
   accagcccga cgagcc 76
<210> 9
   <211> 61
   <212> DNA
   <213> Artificial
<220>
   <223> MLPA Primer
<400> 9
   cctcaagggc acctttgcca cactgagtga gctgcactgt gacaagctgc acgtggatcc 60
   t 61
<210> 10
   <211> 82
   <212> DNA
   <213> Artificial
<220>
   <223> MLPA Primer
<400> 10
   gggcaataat gatacaatgt atcatgcctc tttgcaccat tctaaagaat aacagtgata 60
   atttctgggt taaggcaata gc 82
<210> 11
   <211> 70
   <212> DNA
   <213> Artificial
<220>
   <223> MLPA Primer
<400> 11
   gggaagagga acttctacct acgatggatt tgggttagca tgggctatat cagaatacat 60
   tgcaacaaag 70
<210> 12
   <211> 61
   <212> DNA
   <213> Artificial
<220>
   <223> MLPA Primer
<400> 12
   ggtggtggcc attttttgtc taaccctaac tgagaagggc gtaggcgccg tgcttttgct 60
   c 61
<210> 13
   <211> 46
   <212> DNA
   <213> Artificial
<220>
   <223> MLPA Primer
<400> 13
   aggcctttca ggccgcagga agaggaacgg agcgagtccc cgcgcg 46
<210> 14
   <211> 58
   <212> DNA
   <213> Artificial
<220>
   <223> MLPA Primer
<400> 14
   tctccctggg gaagcatgcc aagctctcgc tgcaggagct gacgtggaag atgagcgt 58
<210> 15
   <211> 77
   <212> DNA
   <213> Artificial
<220>
   <223> MLPA Primer
<400> 15
   ctcgtcgagc tgctcaggtc tttcttttat gtcacggaga ccacgtttca aaagaacagg 60
   ctctttttct acccatg 77
<210> 16
   <211> 19
   <212> DNA
   <213> Artificial
<220>
   <223> MLPA Primer
<400> 16
   accccgccgc gacccatac 19
<210> 17
   <211> 32
   <212> DNA
   <213> Artificial
<220>
   <223> MLPA Primer
<400> 17
   agtctctgtg caacaactta gtggtgtggc ag 32
<210> 18
   <211> 25
   <212> DNA
   <213> Artificial
<220>
   <223> MLPA Primer
<400> 18
   cgaggacgag gacaaggaaa acgat 25
<210> 19
   <211> 29
   <212> DNA
   <213> Artificial
<220>
   <223> MLPA Primer
<400> 19
   attctaggcg catgtgtttc caatagtct 29
<210> 20
   <211> 38
   <212> DNA
   <213> Artificial
<220>
   <223> MLPA Primer
<400> 20
   atgcactagc gcacagagct gcaaacaact atacatga 38
<210> 21
   <211> 41
   <212> DNA
   <213> Artificial
<220>
   <223> MLPA Primer
<400> 21
   aaactttaaa catttatcac atacagcata tggattccca t 41
<210> 22
   <211> 23
   <212> DNA
   <213> Artificial
<220>
   <223> MLPA Primer
<400> 22
   cagctcagag gaggaggatg aaa 23
<210> 23
   <211> 21
   <212> DNA
   <213> Artificial
<220>
   <223> MLPA Primer
<400> 23
   ctttgtacgc acaaccgaag c 21
<210> 24
   <211> 29
   <212> DNA
   <213> Artificial
<220>
   <223> MLPA Primer
<400> 24
   atgcactagc gacctggaca ctgtggact 29
<210> 25
   <211> 35
   <212> DNA
   <213> Artificial
<220>
   <223> MLPA Primer
<400> 25
   tcacctttta aatgtattat aggcgtagtg ttacc 35
<210> 26
   <211> 35
   <212> DNA
   <213> Artificial
<220>
   <223> MLPA Primer
<400> 26
   gatggcaaca aagacaattg tatgacctat gtagc 35
<210> 27
   <211> 44
   <212> DNA
   <213> Artificial
<220>
   <223> MLPA Primer
<400> 27
   cacattcact tttaaattct atataaaacg tgttgtaccc ttcc 44
<210> 28
   <211> 33
   <212> DNA
   <213> Artificial
<220>
   <223> MLPA Primer
<400> 28
   atgcactagc aagatgtgag aaacacacca caa 33
<210> 29
   <211> 30
   <212> DNA
   <213> Artificial
<220>
   <223> MLPA Primer
<400> 29
   acaggttatt tctatgtctt gcagtgaagt 30
<210> 30
   <211> 25
   <212> DNA
   <213> Artificial
<220>
   <223> MLPA Primer
<400> 30
   ttccggttga ccttctatgt cacga 25
<210> 31
   <211> 32
   <212> DNA
   <213> Artificial
<220>
   <223> MLPA Primer
<400> 31
   caacacatac acaacattgt gtgacgttgt gg 32
<210> 32
   <211> 26
   <212> DNA
   <213> Artificial
<220>
   <223> MLPA Primer
<400> 32
   ctaaggtgaa ggctcatggc aagaaa 26
<210> 33
   <211> 25
   <212> DNA
   <213> Artificial
<220>
   <223> MLPA Primer
<400> 33
   caagcgtccc atagactcac cctga 25
<210> 34
   <211> 32
   <212> DNA
   <213> Artificial
<220>
   <223> MLPA Primer
<400> 34
   atttctaata ctttccctaa tctctttctt tc 32
<210> 35
   <211> 38
   <212> DNA
   <213> Artificial
<220>
   <223> MLPA Primer
<400> 35
   tcagttacaa tttatatgca gaaatattta tatgcaga 38
<210> 36
   <211> 37
   <212> DNA
   <213> Artificial
<220>
   <223> SPA Primer
<400> 36
   aggtctgcaa ccaaagattc attaataatc atagatg 37
<210> 37
   <211> 27
   <212> DNA
   <213> Artificial
<220>
   <223> MLPA Primer
<400> 37
   gaaaatgggt tgcaaacatg caaaaag 27
<210> 38
   <211> 16
   <212> DNA
   <213> Artificial
<220>
   <223> MLPA Primer
<400> 38
   gcagcgcacc gggttg 16
<210> 39
   <211> 21
   <212> DNA
   <213> Artificial
<220>
   <223> MLPA Primer
<400> 39
   agcgagaaaa acagcgcgcg g 21
<210> 40
   <211> 18
   <212> DNA
   <213> Artificial
<220>
   <223> MLPA Primer
<400> 40
   gtcagccgcg ggtctctc 18
<210> 41
   <211> 20
   <212> DNA
   <213> Artificial
<220>
   <223> MLPA Primer
<400> 41
   cacagctcag ggaatcgcgc 20
<210> 42
   <211> 20
   <212> DNA
   <213> Artificial
<220>
   <223> MLPA Primer
<400> 42
   acaacgaacg ccgcttcctc 20
<210> 43
   <211> 19
   <212> DNA
   <213> Artificial
<220>
   <223> MLPA Primer
<400> 43
   acctgggctc ctgcgcagc 19
<210> 44
   <211> 25
   <212> DNA
   <213> Artificial
<220>
   <223> MLPA Primer
<400> 44
   aagttcctgc actggctgat gagtg 25
<210> 45
   <211> 24
   <212> DNA
   <213> Artificial
<220>
   <223> MLPA Primer
<400> 45
   gcaacttgct ccagacactc ttcc 24

## Description

### Field of the Invention

The present invention is related to the field of pathogen diagnostics and provides the means for assessing the physical status of a viral, bacterial or any other pathogenic infection in a host, including parasites, yeast, fungi, etc ....

It is in particular directed to the determination of human papilloma virus (HPV) and the application of the assays according to the invention in monitoring the disease progression of HPV related cancers, i.e. in the differentiation between regressive and progressive HPV infected lesions.

Where nearly all cervical cancers (about 99%) are related to HPV, also many anal cancers are caused by the same types of genital HPV that cause cervical cancer. A little less than half of cancers of the vulva are HPV-related. Several other genital cancers (cancers of the penis, vagina, and urethra) and some head and neck cancers (specifically of the tongue and tonsils) may be related to the high-risk types of HPV. Also, a high portion of skin cancers in people with weakened immune systems might be related to this virus.

There is accordingly a need and desire in the field for an HPV assay that provides the means for typing and assessing the physical status of a HPV infection in a host.

### Background to the Invention

Cervical cancer is the second most common cancer in women worldwide. In 2002 there were estimated to be 493,000 new cases and 274,000 deaths as a consequence of this disease worldwide (27).

Human papillomavirus (HPV) plays a causal role in the development of this disease (32). HPV is identified in the majority of cervical cancer cases (39) but still most HPV infected cervical intraepithelial lesions are known to regress spontaneously. The latter implicates that although HPV is a necessary cause, it is not independently able to cause cancer. Individual susceptibility and other factors also play a role in the progression of these intraepithelial lesions to cancer.

In the past years many studies have focused on the identification of markers that can predict the progression of cervical intraepithelial neoplasia (CIN) to cervical cancer and thus may help to discriminate between regressive and progressive HPV infected lesions. These include HPV type, viral load and physical status (5, 18, 19) and gain of telomerase related genes. First, more than 100 types of HPV have been identified of which only a subset (30-40 types) is able to infect the genital tract. Of this subset only 15-18 types are found in cervical cancer and are called high risk (HR) HPV types. The other types are found in more benign disorders such as genital warts and are called low risk (LR) HPV types. Types 16 and 18 are the most prevalent HR-types and are found to be responsible for over 70% of cervical carcinomas (6, 10).

The HR-types 31, 33, and 45 are identified in an additional 10% of the cases (26). Distinction between HPV types can therefore give a risk indication to the development of cancer. Second, if an HR-HPV infection is present in a high viral load it is more likely that this infection will persist (12, 15). A persistent infection in turn, will enhance the likelihood of progression to cancer.

Third, it has been found that part of the HPV genome will be lost upon integration into the host genome. This predominantly concerns the E2 gene but may also include the E1 or L1 gene (8). Several studies have analyzed the most often deleted region but there is a lot of disagreement about the precise position of this deletion. Still it is thought that most of these deletions will cause silencing of the E2 gene which in turn will lead to a consistent overexpression of E6 and E7 genes. These genes act as oncoproteins via degradation of p53 and inactivation of Rb, respectively. It has also been described that integration and the size of the deleted fragment, correlates with severity of the lesion and disease progression (5, 9, 36). Therefore analysis of the physical status of HPV and the size of the deleted fragment can be an indication for progression to cancer.

The last marker concerns gain of the telomerase related genes and is focused on the host instead of the virus (2, 13, 20, 24). Genetic alterations are frequently found in many cancer types (1). By mapping the chromosomal aberrations found in different cancer stages an attempt has been made to identify which aberrations are associated with cancer progression. Since chromosomal aberrations have also been found in premalignant lesions as well as in cervical cancer it seems likely that it plays a causal role in the progression to cancer. One of the aberrations frequently found is a gain of chromosomal arm 3q. This gain appears to be an event which occurs early in carcinogenesis (14, 16, 35) and TERC is 1 of the suggested candidate genes which is located in this region. Another frequently found aberration involves chromosomal arm 5p. In most cases amplification of this region is reported to be associated with progression to cancer (17, 24) but there are also reports implying an association between deletion of 5p and progression to cancer (2).

Several assays are available to analyze one or more of these markers. For example PCR for E2 (3, 37), in some cases combined, with E6 (4), is often used to determine HPV physical status. If both genes are analyzed by a quantitative real-time PCR it can even be used to determine viral load (28). Some other examples are L1 PCR and sequencing for HPV typing; the DIPS (detection of integrated papillomavirus sequences (22)) and APOT (amplification of papillomavirus oncogene transcripts (19)) assay to determine HPV physical status; fluorescence in situ hybridization (FISH), a technique used to analyze chromosomal aberrations as well as to determine HPV type and physical status; and (array)CGH (comparative genomic hybridization) and LOH (loss of heterozygosity) analysis for the screening of chromosomal aberrations.

ANDERSSON ET AL (INTERNATIONAL JOURNAL OF ONCOLOGY, vol. 29, no. 3, September 2006) describe a method to detect p16 expression of the host cell through immunocytochemistry (page 708), the determination of the physical status of HPV by determining E2/E6 mRNA ratio by doing a NASBA assay for these 2 mRNAs, and a determination of viral load and HPV type through real time PCR (see also materials and methods, page 707). p16 is a disease progression marker of cervical cancer (page 706, left column, par. 3-4). D2 detects these 3 features of infection in different assays.

Although with the above described methods associations were found between e.g. the physical status of the virus and the severity of the premalignant lesion and the increased genomic instability in advanced lesions, the methods in most cases were applied as a single marker assay. Furthermore several studies, in cervical carcinomas as well as other HPV related carcinomas, have analyzed (pre)malignant lesions by combining multiple assays. For example Wilting et al. used the L1 PCR, FISH, array CGH, multiplex ligation-dependent amplification assay (MLPA) and reverse transcriptase PCR to extensively analyze cervical lesions and Gagne et al. used L1 PCR, RS-PCR and array CGH for the analysis of anal lesions.

The development of an assay to enable the simultaneous detection of the viral type, viral load, integration status of the virus and genomic instability of the host in one assay would strongly improve the analysis of individual lesions as well as reduce the amount of time and material needed for this analysis.

One such effort in providing an assay for the simultaneous detection of multiple HPV strains in a single reaction is based on the Invader® technology of Third Wave Technologies Inc. (PCT publication WO2005/030041). Compared with said methodology, the assay of the present invention not only allows detecting a plurality of HPV strains, optionally with the co-detection of a human genomic internal control sequence, but also allows a co-determination of the viral integration status, viral load and the genomic instability of the host in a single assay. A further difference between Third Waver and the MLPA of the present invention, is the capability of the present invention to determine up to 40 different kinds of genetic markers (30) instead of 3 to 5.

Where MLPA is typically used to look at small changes in copy number of a gene of interest, as applied in the assays of the present invention, MPLA was surprisingly found to have sufficient discriminating power in revealing a wide range of copy number ratios for a variety of genes in a single reaction. In a single reaction, a mixture of MLPA probes was found to have sufficient discriminating power in revealing, a small range of Copy Number Ratio's (CNR) to determine integration (e.g. to discriminate between 10 and 9 copies E2 to measure 10% integration), a wide range of CNRs for viral load (e.g. to discriminate between 1 to 100 copies E6) and single copy changes for the human genes of interest in determining the genomic instability of the host.

This and other aspects of the present invention will be provided in more detail hereinafter.

### Brief Description of the Drawings

### Figure 1A

### Schematic representation of the different steps in the MLPA assay.

Overview of the HPV-MLPA technology. A pre-amplification is performed with specific primers (fwd = forward primer; rev = reverse primer) for all targets (steps 1). Subsequently, an MLPA reaction is performed with MLPA probes specific for all targets (steps 2, 3, and 4). An MLPA probe consists of two oligonucleotides: one synthetic oligonucleptide and one M13-derived oligonucleotide. The synthetic oligonucleotide contains a universal forward priming site, and the M13-derived oligonucleotide contains a universal reverse priming site. In addition, the M13-derived oligonucleotide contains a unique stuffer sequence. The length of this stuffer sequence is specific for each probe and varies between the different probes. The length of the MLPA probe is the combined length of both oligonucleotides. This length is unique for each probe due to the specific stuffer sequence. The two oligonucleotides hybridize specifically to the target adjacent to each other (step 2). Subsequently, the two oligonucleotides are joined by ligation (step 3) and the ligated oligonucleotides are amplified by one universal primer set (step 4). After amplification, the amplification products can be analysed by, for example, capillary electrophoresis (step 5A; used in our study) or by slab gel analysis (step5B; see (27)). Each MLPA probe can be discerned due to its specific length.

### Figure 1B

Flowchart how to use / interpret the HPV MLPA test.

### Figure 2

### Analysis of viral load = 1 series.

Capillary electrophoresis peak profile for a HPV/human mixture containing 3000 episomal viral copies and 3000 human genome copies, HPV 16 (A) and 18 (B). Standard curve to demonstrate the reliability to determine the % of integration (C). On X-axis % of integrated HPV in different mixtures input DNA (known % integration) and on Y-axis calculated % of integrated HPV based on measured and normalized signal intensities ratio for E2/E6. Graph to illustrate the limited influence of variable copy numbers on the signal intensity ratio of stable targets (D). On the X-axis % of integrated HPV in different mixtures input DNA (variable copy number 16E2.1 and 16E2.2) and on the Y-axis the signals intensity ratio (intensity TERT or TERC/total human signal intensity) for the stable targets.

### Figure 3

Quantitative analysis of HPV in samples with variable viral load. (A) Histogram showing the relation between viral and human signal intensities. The contribution of either all seven human targets (grey bar) or of E6 and E7 (open bar) to the total signal intensity is plotted on the Y-axis. The X-axis shows the copy number ratio (0.1-100) for HPV versus human DNA. Input DNA in all samples is about 10 ng of human DNA. Each bar represents the average + SD of eleven samples. (B,C) Correlation between HPV/human DNA copy number ratio present in the sample and measured signal intensity ratio for E6 (B) or E7 (C) to the signal intensity of all seven human targets. The values are plotted on a double logarithmic scale with the HPV/human DNA copy number ratios (CNRs) on the Y axis and measured signal intensity ratios on the X axis.

### Figure 4

Analysis of E2.2/E6 intensity ratio in series with different viral loads and mixtures with different % of viral integration. In A the comparison of the dot plots for the different mixtures and viral loads. In B the measured E2.2/E6 ratio in the viral load = 100 mixtures before and after a 5-fold dilution, input DNA before dilution 10 ng, after dilution 2 ng. In C-F the measured E2.2/E6 intensity ratio plotted on the X-axis and the % of integrated HPV (input DNA) on the Y-axis for the series with different viral loads. No normalization is performed.

### Figure 5:

Examples of capillary electrophoresis peak profiles for DNA isolated from: A) normal lymphocytes, B) MCF7, C) SiHa, D) HeLa and E) CaSki.

### Figure 6:

Reproducibility of viral load estimations (HPV copies per cell). A) Comparison of the duplicate MLPA measurements and (B) a comparison of the viral load estimations by MLPA and qPCR.

### Figure 7:

Reproducibility of detection of viral integration using E2/E6 ratios. Comparison of the duplicate MLPA measurements for the E2.1/E6 (A) and E2.2/E6 (B) signal intensity ratios.

### Detailed Description of the Invention

This description relates to a multiparameter assay to assess both the physical status of a pathogen (in particular a virus), and the genomic instability of the host, said assay being characterized in that the multiparameter assay is performed in a single reaction compartment.

As used herein, pathogens are defined as any disease-producing microorganism. Pathogens include, but are not limited to, bacteria, viruses, parasites, fungi, yeast, mycoplasma, algae, amoeba, or other microorganisms.

As used herein and contrary to the normal meaning of the term, in the assays according to the present invention 'the physical status of the pathogen' is not limited to the assessment of the genomic integration, but meant to include; typing (identification) the pathogen; analysis of the amount of pathogen present (quantification), and if applicable assessing the genomic integration of said pathogen (characterization); in said sample.

The term "sample" in the present specification and claims is used in its broadest sense. Samples may include fluid (e.g. blood, saliva, cerebral spinal fluid, pleural fluid, milk, lymph, sputum and semen), solid (e. g., stool) or tissue (e.g. cervical tissue) sections taken (isolated) from a subject. In some embodiments it means a suitable quantity of cells or tissue for testing for the physical status of the pathogen, such as a tissue section, blood sample, mouth or cervical swab sample, saliva sample, or other biological fluid sample taken (isolated) from the subject

In one embodiment of the assay according to the invention, the physical status of the pathogen, i.e. virus is determined by the analysis of the viral type, the viral load and the viral integration of the virus, in said sample.

The genomic instability of the host is determined using one or more disease progression marker genes of said host, i.e. any genomic regions/genes known to be associated with an infection of the host with the pathogen of interest.

Changes in copy number, i.e. gain or loss in copy number of said disease progression markers is indicative for the genomic instability of the host, i.e. in its disease progression into cancer.

For example, in determining both the physical status of a HPV infection, and the genomic instability of a human host in case of said HPV infection, the human progression markers are meant to include any genomic regions/genes known to be associated with HPV related cancers, such the chromosome regions 3q, 5p, 20q, 17q, 8q and 1q; in particular the regions 3q and 5p. More in particular the genes within said genomic regions, such as TAF4A RNA polymerase II, TBP-associated factor, Protein kinase C binding protein 1, Nuclear receptor coactivator 6, Splicing factor (CC1.3), Tumor differentially expressed 1, and RAE1 RNA export 1 for chromosome region 20q; such as HER-2/neu (ErbB2) proto-oncogene, the metastasis-suppressor gene nm23, and the BRCA1 gene for chromosome region 17q; TERT on chromosome region 5p and TERC on chromosome region 3q; more preferably TERT on chromosome region 5p and TERC on chromosome region 3q.

As already mentioned hereinbefore, the assays of the present invention are in particular to type and assess the physical status of human papilloma virus (HPV) in a sample; in particular HPV 16 and/or HPV 18. Although HPV has been identified as the primary, cause of cervical cancer, only about 40 of the about 150 different HPV types is capable of infecting the genital tract and only between 13 - 19 of said strains are classified as high-risk viral types for the development of HPV related cancer (Table 0).

**Table 0 HPV classification**

| HPV types | | |
|---|---|---|
| High risk | Probable high risk | Low risk |
| 16 | 26 | 6 |
| 18 | 53 | 11 |
| 31 | 66 | 40 |
| 33 | | 42 |
| 35 | | 43 |
| 39 | | 44 |
| 45 | | 54 |
| 51 | | 61 |
| 52 | | 70 |
| 56 | | 72 |
| 58 | | 81 |
| 59 | | CP6108 |
| 68 | | |
| 73 | | |
| 82 | | |

Accordingly in one aspect of the present invention the HPV types assessed in the methods as provided herein, are selected from HPV types enlisted in Table 0; in particular the high-risk HPV types enlisted in Table 0; more in particular the HPV types HPV 16 and HPV 18 together accounting for as much as 55-85% of the HPV infections.

For said HPV viruses the viral load, typing and integration is determined by the changes in copy numbers of the viral genes that are always present, i.e. independently of the integration status of the virus, and of viral genes that can be partly or completely deleted upon genomic integration of the virus in the human genome. The former can be used to type the HPV virus and to determine the load of the virus in the sample. The latter can be used to type the virus and to determine the integration status of the virus in the sample. HPV genes known to be present independently of the integration status of said virus into the human genome, include E6 and E7. HPV genes know to be partly or completely deleted upon genomic integration, include E1, E2, E4, E5, L1 and L2. As shown in the examples hereinafter, in a particular embodiment, the viral genes used to determine the viral load, typing and integration consist of the viral genes E2, E6 and E7.

In said embodiment, the sample means a suitable quantity of cells or tissue, e. g., cervical cells, or cervical tissue, for testing for the presence of any HPV-related cancer, like cervical cancer. The sample can take the form of a biopsy, a smear, or a swab containing cells.

As provided in more details in the examples hereinafter, the viral load is determined as the ratio between the copy numbers of the viral genes as defined hereinbefore, i.e. of the viral genes that are always present independently of the integration status of the virus; in particular of any one of E6 or E7 and of the copy numbers of one or more reference marker genes of the host; more in particular as the average of the gene copies of E6 and E7 vis-à-vis the gene copies of □-globin, MSH2, TERT and TERC; in particular of □-globin and MSH2 in said host.

Again provided in more details in the examples hereinafter, the viral integration is determined by the ratio in copy numbers of viral genes that can be partly or completely deleted upon genomic integration of the virus in the human genome and copy numbers of a viral gene that is always present independently of the integration status of the virus. In a particular embodiment the ratio between the viral gene copies of one of the viral gene(s) selected from E1, E2, E4, E5, L1, and L2; with E6. In a further embodiment as the ratio between the viral gene copies of one of the viral gene(s) selected from E1, E2, E4, E5, L1, and L2; with E7. More in particular as the ratio between the viral gene copies of E2 with E6 and/or E7; alternatively expressed as the average of the ratios between, the copy numbers of the viral genes E2 and E6, and of the viral genes E2 and E7.

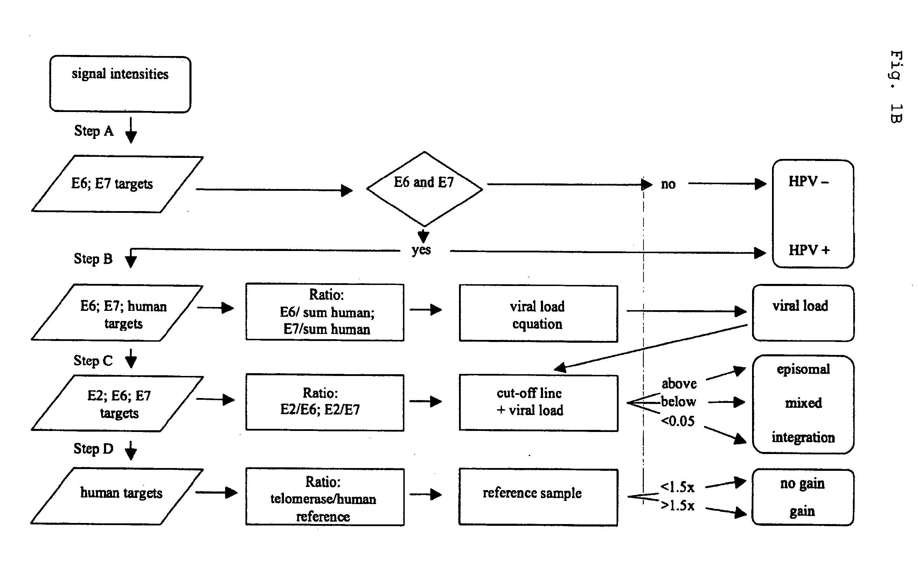

As provided in more details in the examples hereinafter, in an even further embodiment the ratio in copy numbers of E2 to E6 and/or of E2 to E7 is normalised vis-à-vis the ratio in copy number of said genes in a reference sample such as an episomal sample, i.e. a sample wherein the virus is not integrated into the host genome and accordingly represents the fraction of non-integrated viral genome. It is in the examples hereinafter, referred to as the copy number ratio (CNR). Based on said ratio one can determine a viral load equation and cut off line taking into account the calculated viral load and reference sample. A text chart representing the different steps is enclosed in Figure 1B.

As already mentioned hereinbefore, in typing the pathogen in the sample, one determines the presence of copy numbers of genes that are always present, i.e. independently of the integration status of the virus. In typing a HPV virus in an assay according to the invention, the genes are in particular selected from E6 or E7. Optionally, the viral genes that can be partly or completely deleted upon genomic integration of the virus in the human genome can be used in combination with the genes that are always present in the typing the pathogen in the sample. Thus in a further embodiment when typing a HPV virus in an assay, one may optionally determine the presence of the viral E2 gene(s) in said sample.

As used herein, the TERT, TERC, E2, E6, E7 and host (human)' reference gene (i.e. □-globin or MSH2) presence is determined at the nucleic acid or protein level; in particular at the nucleic acid level.

In a particular embodiment of the present invention, the expression of TERT, TERC, E2, E6, E7 and the host (human) reference gene(s) is determined using multiplex ligation-dependent probes (MLPA probes) in a multiplex ligation-dependent amplification assay (MLPA).

In said MLPA assay, the MLPA probes comprise a target specific hybridizing oligonucleotide sequence; and include at least 2 E2 MLPA probes, at least 1 E6 MLPA probe, at least 1 E7 MLPA probe, at least 1 TERT MLPA probe, at least 1 TERC MLPA probe and at least 1 MLPA probe for a human reference gene. In a particular embodiment of said MLPA assay, the MLPA probes include one E2 MLPA probe, one E6 MLPA probe or one E7 MLPA probe, one TERT MLPA probe, one TERC MLPA probe and at least one MLPA probe for a human reference gene, such as □-globin and MSH2.

In one embodiment of the present invention, the target specific hybridizing oligonucleotide sequence(s) used in the aforementioned MLPA probes are selected from the group represented in table 1; in particular consist of the hybridizing oligonucleotide sequences represented in table 1.

In principle any host reference gene can be used in the assays according to the invention, and include for example □-globin and MSH2 or any other human gene located in a genomic region known not being amplified or modified (e.g. deletions, mutations, amplifications, ... ) in cancer cells, i.e. genes located in a quiet, stable genetic domain.

In a further embodiment of the MLPA assay a pre-amplification reaction is performed to increase the nucleic acids present in the sample; in particular an asymmetric PCR reaction is used for said pre-amplification; more in particular using the primers represented in table 2.

It is also an aspect of the present invention to provide the use of an assay as described herein, to type and assess the physical status of a virus in a sample; in particular in monitoring the disease progression of HPV related cancer, i.e. in the differentiation between regressive and progressive HPV infected lesions.

In a further aspect the present invention provides a kit comprising MLPA probes as defined herein, optionally with pre-amplification primers such as represented in table 2.

This invention will be better understood by reference to the Experimental Details that follow, but those skilled in the art will readily appreciate that these are only illustrative of the invention as described more fully in the claims that follow thereafter. Additionally, throughout this application, various publications are cited.

### EXAMPLES

The following examples illustrate the invention. Other embodiments will occur to the person skilled in the art in light of these examples.

### Materials and Methods

### Cell lines

Human uterine cervical cancer cell lines (CaSki, SiHa and HeLa), human breast cancer cell lines (MCF7 and T47D), the human intestinal epithelial cell line CaCo-2 and the human immortalized non-tumorigenic keratinocyte cell line HaCaT were obtained from the American Type Culture Collection (ATCC, Manassas, VA, USA). All cell lines were grown according to the suppliers' recommendations and DNA was extracted using the QIAamp DNA Mini Kit (Qiagen, Hilden, Germany) according to protocol.

### Uterine cervical tissue samples

Sixty-seven frozen cervical specimens including 7 normal ectocervical tissues, 20 normal ectocervical epithelia adjacent to (pre)neoplastic lesions, 10 CIN1 lesions/condylomata, 13 CIN2/3 lesions and 17 squamous cell carcinomas (SCCs) were obtained from the Tissue Bank of the University of Liege. The samples contained between 30% and 95% (pre)malignant cells. DNA was extracted from each sample by using the NucleoSpin Tissue kit (Macherey-Nagel, Dü ren, Germany) according to the manufacturer's instructions. The project protocol was approved by the Medical Ethics Committee of the University Hospital of Liege.

### Design and preparation of pre-amplification primers and MLPA probes

For the design of the pre-amplification primers and the hybridizing part of the probes, sequence information available from public databases was used, including the Nucleotide database from the National Center of Biotechnology Information (NCBI; http://www.ncbi.nlm.nih.gov). In addition to the BLAST (Basic Local Alignment en Search Tool) analysis program (NCBI), BioEdit (http://www.mbio.ncsu.edu/ BioEdit / BioEdit.html) was used for sequence alignments, and Primer3 design software (Primer3 v 0.4.0; http://frodo.wi.mit.edu/) for primer design.

To identify type-specific HPV16 and 18 regions and for the design of the pre-amplification primers and the hybridizing part of the MLPA probes **(Table 1)**, alignments were performed with the sequences of all HPV types. All primers and probes were evaluated by performing a BLAST analysis against the NCBI database and were approved when no mismatch was found for all known viral subtype and variant sequences within the critical regions of the pre-amplification primers (i.e. no mismatch at the 3' end of a primer) and MLPA probes (i.e. no mismatch within 5 nt from the ligation site). A similar approach was chosen for the design of the pre-amplification primers and MLPA probes for the human targets, i.e. □-globin, MSH2, TERC, and TERT.

For each target 4 forward and 4 reverse pre-amplification primers were designed which were tested in all possible combinations, resulting in 16 primer combinations per target and a total of 240 primer combinations. For each target the specificity of all combinations was determined and 4 specific combinations with the strongest signal intensity were selected for a second test.

**Table 1: MLPA probes**

| Target | Gene | Sequence 5'-3' | Position (nt) | Reference |
|---|---|---|---|---|
| HPV16E2.1 | E2 (E4) | | 3442-3502 | AY686580 |
| HPV16E2.2 | E2 | | 2769-2841 | AY686580 |
| HPV16E6 | E6 | | 180-255 | AY686580 |
| HPV16E7 | E7 | | 686-749 | AY686580 |
| HPV18E2.1 | E2 (E4) | | 3582-3648 | AY262282 |
| HPV18E2.2 | E2 | | 3230-3299 | AY262282 |
| HPV18E6 | E6 | | 110-167 | AY262282 |
| HPV18E7 | E7 | | 676-751 | AY262282 |
| □-globin_a | □-globin | | 866814-866874 | NW_925006 |
| □-globin_b | □-globin | | 866142-866223 | NW_925006 |
| MSH2 | MSH2 | | 2318-2387 | NM_000251 |
| TERC_a | TERC | | 75977912-75977972 | NT_005612 |
| TERC_b | TERC | | 75977602-75977647 | NT_005612 |
| TERT_a | TERT | | 3452-3509 | DQ264729 |
| TERT_c | TERT | | 14314-14386 | DQ264729 |

**Table 2: Primers for pre-amplification**

| Name | Reference | Sequence 5'-3' | Location | Fragment size |
|---|---|---|---|---|
| HPV16E2.1 | AY686580 | 5'-ACCCCGCCGCGACCCATAC | 3408-3426 | 129 bp |
| | | 5'-AGTCTCTGTGCAACAACTTAGTGGTGTGGCAG | 3505-3536 | |
| HPV16E2.2 | AY686580 | 5'-CGAGGACGAGGACAAGGAAAACGAT | 2733-2757 | 141 bp |
| | | 5'-ATTCTAGGCGCATGTGTTTCCAATAGTCT | 2845-2873 | |
| HPV16E6 | AY686580 | 5'-ATGCACTAGCGCACAGAGCTGCAAACAACTATACATGA | 140-177 | 161 bp |
| | | 5'-AAACTTTAAACATTTATCACATACAGCATATGGATTCCCAT | 270-310 | |
| HPV16E7 | AY686580 | 5'-CAGCTCAGAGGAGGAGGATGAAA | 651-673 | 123 bp |
| | | 5'-CTTTGTACGCACAACCGAAGC | 753-773 | |
| HPV18E2.1 | AY262282 | 5'-ATGCACTAGCGACCTGGACACTGTGGACT | 3558-3577 | 142 bp |
| | | 5'-TCACCTTTTAAATGTATTATAGGCGTAGTGTTACC | 3666-3700 | |
| HPV18E2.2 | AY262282 | 5'-GATGGCAACAAAGACAATTGTATGACCTATGTAGC | 3192-3226 | 166 bp |
| | | 5'-CACATTCACTTTTAAATTCTATATAAAACGTGTTGTACCCTTCC | 3314-3357 | |
| HPV18E6 | AY262282 | 5'-ATGCACTAGCAAGATGTGAGAAACACACCACAA | 78-100 | 123 bp |
| | | 5'-ACAGGTTATTTCTATGTCTTGCAGTGAAGT | 171-200 | |
| HPV18E7 | AY262282 | 5'-TTCCGGTTGACCTTCTATGTCACGA | 651-675 | 136 bp |
| | | 5'-CAACACATACACAACATTGTGTGACGTTGTGG | 755-786 | |
| □-globin-a 11p15.5 | NW_925006 | 5'-CTAAGGTGAAGGCTCATGGCAAGAAA | 866916-866941 | 160 bp |
| | | 5'-CAAGCGTCCCATAGACTCACCCTGA | 866806-866782 | |
| □-globin-b 11p15.5 | NW_925006 | 5'-ATTTCTAATACTTTCCCTAATCTCTTTCTTTC | 866225-866256 | 159 bp |
| | | 5'-TCAGTTACAATTTATATGCAGAAATATTTATATGCAGA | 866135-866098 | |
| MSH2 2p21 | NM_000251 | 5'-AGGTCTGCAACCAAAGATTCATTAATAATCATAGATG | 2277-2313 | 145 bp |
| | | 5'-GAAAATGGGTTGCAAACATGCAAAAAG | 2395-2421 | |
| TERC_a 3q26 | NT_005612 | 5'-GCAGCGCACCGGGTTG | 75977993-75978008 | 119bp |
| | | 5'-AGCGAGAAAAACAGCGCGCGG | 75977890-75977910 | |
| TERC_b 3q26 | NT_005612 | 5'-GTCAGCCGCGGGTCTCTC | 75977667-75977684 | 105 bp |
| | | 5'-CACAGCTCAGGGAATCGCGC | 75977580-75977599 | |
| TERT_a 5p15.33 | DQ264729 | 5'-ACAACGAACGCCGCTTCCTC | 3413-3432 | 131 bp |
| | | 5'-ACCTGGGCTCCTGCGCAGC | 3525-3543 | |
| TERT_c 5p 15.33 | DQ264729 | 5'-AAGTTCCTGCACTGGCTGATGAGTG | 14284-14308 | 127 bp |
| | | 5'-GCAACTTGCTCCAGACACTCTTCC | 14387-14410 | |

This test was to determine the most sensitive primer combination through the use of 5 dilutions of DNA i.e. 5 ng; 500 pg; 50 pg; 5 pg and 500 fg in the 20 µl PCR reaction mixture for the human targets and 12.5 fg; 1.25 fg; 125 ag; 12.5 ag and 1.25 ag for the HPV targets. The combination with the highest sensitivity and specificity was selected for each target and is presented in **Table 2**. The primers were designed to produce pre-amplification fragments with a maximum of 166 bp in length.

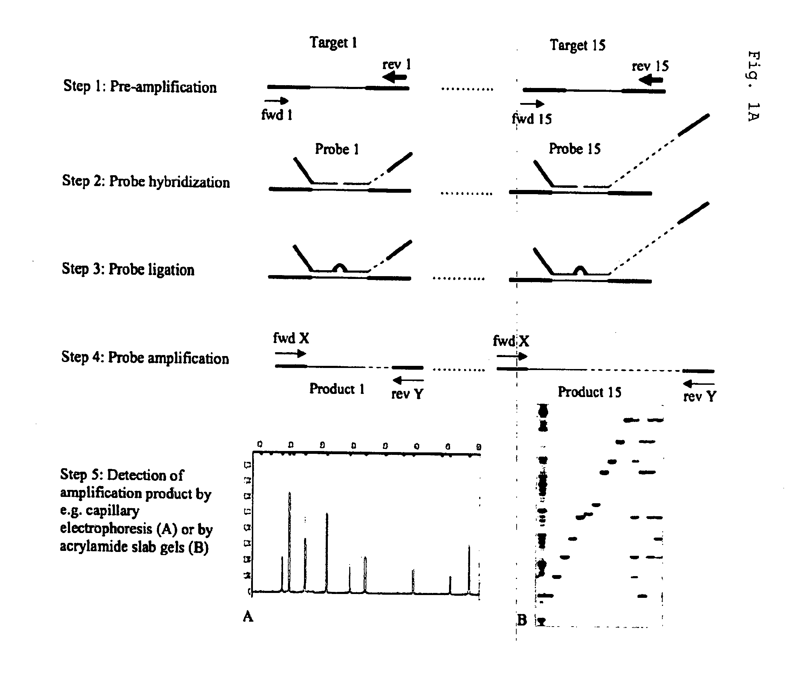

Each MLPA probe set consists of one short synthetic oligonucleotide and one, phage M13-derived, long oligonucleotide (see figure 1A), and gives rise to an amplification product of unique size between 129 and 488 bp. The phage M13-derived, long oligonucleotides were prepared as previously described (30) and the short synthetic oligonucleotides and pre-amplification primers were synthesized by Biolegio (Malden, The Netherlands). For storage the oligonucleotides were diluted in TE-buffer (10mM Tris-HCl pH 8.0, 1mM EDTA) to a concentration of 100 µM, which was used as stock solution.

### Pre-amplification

A multiplex pre-amplification PCR was performed using the Qiagen Multiplex PCR kit (Qiagen, Hilden, Germany). A 20 µl reaction mixture contained Qiagen mastermix (MgCl2 final concentration 3 mM; dNTPs and HotstarTaq DNA polymerase), multiplex primer mix (final concentration 20 nM for each forward primer and 200 nM for each reverse primer), and 10 ng of sample DNA. Amplification was performed on a Biometra T1 Thermocycler (Biometra, Göttingen, Germany) as follows: 15 min at 95°C, followed by 20 cycles of each 30 sec at 94 °C, 90 sec at 55 °C, and 90 sec at 72°C, followed by a 10 min extended elongation at 72°C.

### MLPA analysis

The MLPA analysis was performed as described by Schouten et al (30) with minor modifications. The pre-amplified product was diluted 5 times using sterile water, after which 2 µl was mixed with 1.5 µl MLPA-buffer (1.5 M KC1, 300 mM Tris-HCl pH 8.5, 1 mM EDTA; MRC-Holland, Amsterdam, the Netherlands), 1.5 µl probemix (3 fmol of each synthetic probe oligonucleotide and 1.5 fmol of each M13-derived oligonucleotide in TE) and 3 µl sterile water. After a 5 min denaturation step at 98 °C in a Biometra T1 Thermocycler with a heated lid, the mixture was incubated for 16h at 60 °C. For ligation this mixture was diluted to 40 µl with ligation buffer (2.6 mM MgCl2, 5 mM Tris-HCl pH 8.5, 0.013% non-ionic detergents, 0.2 mM nicotinamide adenine dinucleotide) containing 1U heat-stable Ligase-65 enzyme (MRC-Holland, Amsterdam, the Netherlands) and incubated at 54 °C for 15 min, followed by ligase inactivation at 98 °C for 5 min. Four µl of this mixture was added to 16 µl of PCR mixture containing dNTPs (2 mM each, Fermentas, St. Leon-Rot, Germany), 1 U Taq-polymerase (MRC-Holland, Amsterdam, the Netherlands), 1x PCR buffer (50 mM KCl, 10 mM Tris-HCl pH 8.5, 1.6 mM MgCl2) and 4 pmol of the two MLPA-PCR primers each, with the forward primer 5'-GTGGCAGGGCGCTACGAACAA-3' labeled with carboxyfluorescein (FAM), and the reverse primer 5'-GGACGCGCCAGCAAGATCCAATCTAGA-3'. Amplification was performed on a Biometra T1 Thermocycler as follows: an initial cycle of 2 min at 95 °C, followed by 33 cycles of 30 sec at 94 °C, 30 sec at 60 °C and 1 min at 72 °C, followed by a 10 min extended elongation at 72 °C. MLPA buffers and enzymes were obtained from MRC-Holland (Amsterdam, the Netherlands).

### Analysis of PCR products

Amplified FAM labeled MLPA products were analyzed by electrophoresis on an ABI3730 genetic analyzer (Applied Biosystems, Foster City, CA, USA). One µl of 20x diluted amplified MLPA products was mixed with 8.5 µl of deionized formamide and 0.5 µl of GeneScan-600 LIZ® size standard (Applied Biosystems, Foster City, CA, USA) and run in GeneScan mode. All analyses were done according to the manufacturers' instructions. Electropherograms were analyzed by GeneMarker software (Softgenetics, State College, PA, USA), and peak height data were exported to Excel files for calculation of ratios. E6 and E7 loads were estimated by determining the ratio between E6 or E7 and the seven human targets named hereinbefore (see table 1), i.e. □-globin (2x), MSH2, TERT (2x), and TERC (2x), and using this ratio in the equations as shown in Table 3 below.

*Plasmid model systems for viral integration and viral load* To determine what percentage of integrated HPV can be detected reliably in a background of episomal HPV and vice versa, several mixtures of HPV plasmids, mimicking either integrated or episomal HPV were used. These model systems were also used to determine the lowest concentration of HPV that can be detected in a background of human DNA, and conversely at which concentration of HPV human targets can still be detected.

### HPV16 and 18 plasmids

For each of the two HPV types two plasmids were used for the model systems. One plasmid containing all targets and thereby mimicking episomal HPV and one plasmid with a deletion of at least one of the targets resembling the loss of sequences as a consequence of HPV insertion into the human genome. This latter plasmid will be used to mimic integrated HPV.

Two synthetic plasmids were constructed for HPV16. A synthetic construct was designed containing the target sequences for HPV16 in the following order: 16E2.2, 16E2.1, the reverse complementary target sequence for 16E7 and the reverse complementary target sequence for 16E6. The HPV16 plasmid containing all targets and thereby mimicking the episomal HPV, was made by annealing 28 overlapping oligonucleotides (Biolegio, Malden, The Netherlands), designed with DNAworks (27), covering the entire construct sequence, using the Phusion High-Fidelity PCR Master Mix (Finnzymes, Espoo, Finland).

For the HPV16 plasmid lacking E2, which mimics integrated HPV, only the 16 oligonucleotides representing the 3'-half were used. Initial annealing was performed on a Biometra T1 Thermocycler as follows: an initial cycle of 30 sec at 98 °C, followed by 15 cycles of 10 sec at 98 °C, 30 sec at 60 °C and 90 sec at 72 °C, followed by a 10 min extended elongation at 72 °C. Following initial annealing the constructs were amplified, using only the first and last oligonucleotide, as follows: an initial cycle of 30 sec at 98 °C, followed by 30 cycles of 10 sec at 98 °C, 30 sec at 60 °C and 90 sec at 72 °C, followed by a 10 min extended elongation at 72 °C. The PCR reaction was applied according to the manufacturers' instructions (Finnzymes, Espoo, Finland) in a final volume of 50 µl. The oligonucleotides were used as both primer and templated DNA and a total of 5 pmol of each oligonucleotide was used for each reaction. The constructs were ligated into the pGem-T Easy vector (Promega, Madison, WI, USA) according to manufacturers' instructions. DNA was extracted using The Wizard® Plus SV Minipreps DNA Purification System (Promega, Madison, WI, USA) according to protocol.

The two HPV18 plasmids used in this study were described previously (5, 31). The first plasmid was described to contain a 7.8 kb HPV18 construct and was found to be positive for all 4 HPV18 targets. This plasmid was used to mimic episomal HPV18. The second plasmid was described to contain a 6.9 kb construct. This plasmid was shown to lack 900 bp including E6 and was therefore used to mimic integrated HPV18.

### Reliability of integrated HPV quantification

To determine what percentage of integrated HPV can reliably be detected in a background of episomal HPV and vice versa, several combinations of the above mentioned HPV plasmids, representing either integrated or episomal HPV, were made varying from 100% episomal HPV i.e. a E2/E6 or E2/E7 copy number ratio (CNR) being 1, to 100% integrated HPV with an E2/E6 or E2/E7 CNR being 0.

The CNR value as used in this context, accordingly represent the fraction of episomal HPV and is determined as the ratio in gene copy number presence of E2 to E6 or E7 normalized against the ratio in gene copy number presence of E2 to E6 or E7 in a sample representing episomal HPV.

This was achieved by mixing episomal HPV and integrated HPV to obtain samples with E2/E6 CNRs of 1, 0.9, 0.8 to 0.1 and 0. Each sample contained approximately 3,000 copies of plasmid DNA (37 fg of the HPV 18 plasmids or 12 fg of the HPV 16 plasmids, based on the length of each plasmid) in a background of 10 ng (3,000 copies) of normal human DNA (Promega, Madison, USA). Consequently the HPV (3,000 copies) /human DNA (3,000 copies) Copy Number Ratio (CNR) in these mixtures is 1.

### Reliability of HPV and human DNA copy number estimations

To determine the lowest concentration of HPV that can be detected in a background of human DNA, and conversely to determine at which concentration of HPV the human targets can still be detected, variants of the above described HPV16 integration series were designed, in that the HPV/human DNA CNR varied from 0.1-100. All series were designed to have the same distribution in viral integration status with E2/E6 CNRs varying from 1.0 to 0. Three series were designed to contain more HPV copies than human copies with 300,000, 150,000 or 30,000 copies of HPV, in a background of 3,000 copies of human DNA (HPV/human DNA CNR being 100, 50, and 10, respectively). A fourth series was designed to contain less HPV copies than human DNA copies, with 300 copies of HPV DNA in a background of 3,000 human copies (HPV/human DNA CNR being 0.1).

Furthermore, the influence of the amount of total DNA on the outcome of the experiments was tested. For this purpose the initial integration series was tested with 100 ng instead of 10 ng of total DNA. Also the series with a HPV/human DNA CNR of 100 was tested with 2 ng instead of 10 ng of total DNA.

### Quantification of integration and viral load

Calculation of viral load (defined here as HPV copies per D-globin gene copy) **and** percentage of HPV integration is based on the intensity (peak height) ratio of the probe signals in the capillary electrophoresis profile. The viral load is estimated as the average ratio of the signal intensity of E6 and E7 to the total signal intensity of all seven human targets.

As mentioned hereinbefore, when determining the viral integration, the fraction (percentage) of episomal copies is calculated from the E2/E6 signal intensity ratio (see CNR values above), and as a result the percentage of integrated copies equals 100% minus the percentage of episomal copies. For the plasmid model system with a HPV/human DNA CNR of 1 a standard integration curve was made. For this the known percentage of integration in the prepared mixture was plotted against the percentage of integrated HPV measured as the HPV16 E2.2/E6 or the HPV18 E2.2/E6 signal intensity ration measured with this MLPA assay.

In this setting, and in order to reduce the intersample variability, the measured E2/E6 signal intensity ratio was normalized against the average measured ratio E2/E6 for the reference samples with E2/E6 ratios ranging from 0 to 1. This average measured ratio E2/E6 theoretically corresponds to the reference value for a mixture containing 50% episomal and 50% integrated virus.

Consequently, for this study the measured percentage of integration was calculated by: (1 - (0.5 x ( measured intensity ratio E2/E6) / (the average measured ratio E2/E6)) x 100%.

For the other series of the plasmid model system with HPV/human DNA CNRs of 10, 50 and 100, similar standard integration curve were made.

### Analysis of viral status in patient samples

All samples were analyzed in duplicate and when the viral load in a sample was determined to be less than 1 copy per cell the assay was repeated with a primer and probe mix containing only HPV targets, thus without primers and probes for the human targets. When the viral load in a sample was more than 50 copies per cell the sample was diluted with normal human DNA to decrease the viral load and the diluted sample was analyzed again.

Due to experimental variability a reference series was included in each experiment. This series consisted of 5 mixtures of plasmids and human DNA as described above, mimicking HPV16 samples with viral loads of 2, 5, 10, 20, and 40 copies per cell of which 30% was integrated. In each experiment the reference series was run in duplicate and the subsequent 16E2.1/E6 or 16E2.2/E6 ratio was plotted against the viral load to determine the viral integration cut-off value for different viral loads. Patient samples with a ratio above the cut-off line were scored as episomal and samples with a ratio below the cut-off line were scored as mixed or integrated. This results in a classification of episomal HPV when less than 30% of the virus in a sample shows integration, mixed (i.e. episomal and integrated) HPV when between 30% and 95% of the virus shows integration, and integrated HPV when more than 95% of the virus shows integration.

The specificity of the primer and probe mix for HPV16 and 18 was evaluated by tests on HPV16 or 18 plasmid model systems, cell lines containing HPV16 or 18, and clinical samples containing closely related HPV types, i.e. HPV31, 33 and 45 (data not shown), as determined with the PapilloCheck (*infra*) (Greiner Bio-One GmbH, Frickenhausen, Germany).

### Quantitative PCR (qPCR)

Viral load for HeLa was estimated by qPCR for HPV18 using the pre-amplification primers for 18E6 in combination with LC-green (Idaho Technology Inc., Salt Lake City, Utah, USA). The qPCR reactions were performed on a Rotor-Gene 6000 (Corbett Life Science, Sydney, Australia) as follows: 2 min at 95 °C, followed by 45 cycles of 30 sec at 94 °C, 90 sec at 55 °C, and 90 sec at 72 °C, followed by a 10 min extended elongation at 72°C. The standard curve was obtained by amplification of a 5-fold dilution series of 6,000,000 to 1,920 copies of the HPV18 plasmid in a background of 10 ng of normal human DNA (Promega, Madison, USA) per reaction.

Viral load for HPV16 in tissue samples and the SiHa and CaSki cell cultures was estimated by qPCR using the previously described primers and probes (Peitsaro P. et al., 2002) for 16E6 (Biolegio, Malden, The Netherlands) in a 20 µl PCR mixture containing dNTPs (2 mM each, Fermentas, St. Leon-Rot, Germany), 1 U Taq-polymerase (MRC-Holland, Amsterdam, the Netherlands), 1x PCR buffer (50 mM KCl, 10 mM Tris-HCl pH 8.5, 1.6 mM MgCl2), 3 pmol of each primer and 1 pmol of probe. The qPCR reactions were performed in a Rotor-Gene 6000 real-time system (Corbett Life Science, Sydney, Australia) as follows: 2 min at 95 °C, followed by 50 cycles of 30 sec at 94°C, 90 sec at 55 °C, and 90 sec at 72 °C, followed by a 10 min extended elongation at 72°C. The standard curve was obtained by amplification of a 10-fold dilution series of 5,000,000 to 500 copies of the HPV16 plasmid.

The threshold cycle values for both HPV16 E6 and E2 standard curves were plotted against the log of the copy number over the entire range of dilutions and revealed a linear relationship. These standard curves were used to estimate the average HPV copy number in the individual samples based on duplicate measurements. Viral loads were calculated based on the assumption that the samples have a diploid DNA content and a DNA mass of 7.8 pg per diploid cell (39). The physical status of the virus was determined on basis of the copy number ratio between E2 and E6. Samples were classified as integrated when no fluorescence was detected for E2 and the ratio was 0, mixed for ratios between 0 and 0.5, and episomal for ratios above 0.5 as previously described (40).

### Fluorescence in situ hybridization (FISH)

FISH analysis was performed as previously described (15) using a Vysis probe set (Vysis, Abbott Molecular, Des Plaines, IL, USA) consisting of: a probe for chromosome 3 centromere (CEP3) labeled with the fluorescent dye Spectrum Aqua (SA), a 3q26-specific BAC clone containing the TERC gene labeled with Spectrum Orange (SO) and a 5p15-specific BAC clone containing the TERT gene labeled with Spectrum Green (SG).

Depending on the sample analysed, sample preparation was as follows;

For the different HPV infected cell lines (*supra*), ethanol fixed cells from said cell lines were spotted on glass slides, pretreated by pepsin (100 µg/ml 0.01 N HCl), and dehydrated in an ethanol series. Probes (dissolved in 70% formamide, 2X SSC, 10% dextransulphate) and cellular DNA were simultaneously denatured for 3 min at 80°C and hybridized at 37°C overnight. Slides were washed in 2X SSC (42°C, 2x 5 minutes) and 0.1% Triton X100 in 2X SSC (42°C, 2x 5 minutes), followed by dehydration in an ethanol series, counterstaining with 4',6-diamidino-2-phenylindole (DAPI) and embedding in an Vecta shield (Vector Laboratories, Burlingame, Ca, USA).

For the tissue samples, 4 µm thick fresh frozen tissue sections were heated on glass slides for 20 minutes at 80 °C to improve adherence, pretreated with pepsin (Pepsin from porcine gastric mucosa; 100 µg/ml in 0.01 N HCl, 800-2500 units/mg protein; Sigma Chemical Co., St. Louis, MO, USA), post-fixed in 1% formaldehyde in PBS, and dehydrated in an ascending ethanol series. Probes were dissolved in 70% formamide, 2x saline-sodium citrate pH7.4 (SSC), and 10% dextran sulphate) and simultaneously denatured with the cellular DNA for 3 min at 80 °C and hybridized at 37 °C overnight. For the chromosomal targets the slides were then washed in 2x SSC (42°C, 2x 5 minutes) and 0.1% Triton X-100 in 2x SSC (42°C, 2x 5 minutes), followed by dehydration in an ethanol series, nuclear counterstaining with 0.5 ng/µl 4',6-diamidino-2-phenylindole (DAPI, Sigma Chemical Co.) and embedding in Vectashield (Vector Laboratories, Burlingame, CA, USA). For the HPV targets the slides were washed in 2x SSC (42°C, 2x 5 minutes) and incubated for 30 min at 37°C with mouse anti-digoxigenin (1:2,000 Sigma Chemical Co.), then 30 min at 37°C with a peroxidase-conjugated rabbit antimouse Ig (1:100 DAKO A/S Glostrup, Denmark) and finally a peroxidase-conjugated swine anti-rabbit Ig (1:100 DAKO) for 30 min at 37°C. After washing in PBS/0.05% Tween-20, the tyramide signal amplification reaction was carried out under a coverslip by applying 50 µl rhodamine-labeled tyramide in PBS (1:500 diluted from a 1 mg/ml stock solution in ethanol) containing 0.1 M imidazole, pH 7.6, and 0.001% H2O2 for 10 min at 37°C. The slides were washed in PBS containing 0.05% Tween-20 (Janssen Chimica, Beerse, Belgium), dehydrated in an ascending ethanol series and mounted in Vectashield (Vector Laboratories) containing 0.5 ng/µl DAPI (Sigma Chemical Co.).

Images were acquired using a Leica DMRXA microscope (Leica, Wetzlar, Germany) equipped with custom optical filters for DAPI, SA, SG, and SO (Chroma Technologies, Brattleboro, VT, USA) with a x40 Plan Apo (NA 1.20) objective. The microscope was connected to a digital black and whit CCD camera (Metasystems Image Pro System, Sandhausen, Germany). To obtain an average copy number for the TERC and TERT targets, the FISH spots were counted in a total of 50 interphase cells per cell line.

### PapilloCheck® Assay

The PapilloCheck® HPV-Screen DNA-chip (Greiner, Frickenhausen, Germany) was used for the qualitative detection and differentiation between 24 types of genital HPV (18 high-risk and 6 low-risk). HPV genotyping was performed as previously described by Jones et al. (41). Briefly, for each test at least 40 ng of DNA was used and a 350 bp fragment of the HPV E1 gene was amplified using a multiplex PCR with type-specific primers. An internal PCR control targeting a fragment of the human housekeeping gene ADAT1 was included in each run to avoid false negative results. PCR fragments were fluorescence-labelled with Cy5 and hybridized to specific probes on the PapilloCheck DNA chip. The amplification level was determined by the binding of PCR products to five control spots and their subsequent signal intensity on the chip. Following hybridization and subsequent washing steps, the chip was scanned at excitation wavelengths of 532 and 635 nm.

### APOT / DIPS Assay

DNA was isolated from formalin-fixed and paraffin-embedded material that was matching with 13 frozen tissue samples (*supra*)**.** The samples were analyzed for viral integration by amplification of papillomavirus oncogene transcripts (APOT) analysis on RNA, and detection of integrated papillomavirus sequences (DIPS) analysis on DNA using standard procedures (18, 21, 37).

### Results

### MLPA primer and probe design

HPV probes were developed to assess HPV type, load and physical status (see Table 1). Since the E6 and E7 genes are nearly always present in all HPV related lesions, regardless of the physical status, probes against these genes were designed for typing of HPV 16 and 18. For the detection of the physical status two E2 probe sets per HPV type were developed, which localize in the sequences most frequently deleted upon integration into the human genome. The distance between the two E2 target sequences is 601 bp and 283 bp for HPV 16 and 18, respectively. For the estimation of viral load seven human probe sets were developed recognizing target sequences in the □-globin (two loci), the MSH2, the TERT (two loci) and the TERC gene (two loci). The □-globin gene was included in this assay since it has previously been described as a reference to determine viral load in real-time PCR (19). MSH2 was selected as a reference since chromosome 2p numbers remain relatively stable in HPV related cancers (16, 23, 36). In addition, four probe sets were designed to target sequences within TERC (3q26) and TERT (5p15), which were also used to assess gain of the telomerase associated genes. An overview of the used target sequences is provided in Table 1.

The pre-amplification primers were designed to anneal as close as possible to the 5' and the 3' end of the above mentioned probe targets. Of the 240 initially designed primer combinations the 15 combinations that were selected based on highest sensitivity and specificity are presented in Table 2.

### Plasmid model systems for viral integration and viral load

Plasmid model systems representing different viral and human CNRs were prepared and analyzed by MLPA. The mixtures containing 3,000 human as well as episomal HPV genome copies represented equal copy numbers for human and viral targets and the viral load is considered 1. The capillary electrophoresis peak profiles for both HPV 16 and 18 showed signals for all 11 targets, i.e. 7 human and 4 type specific viral targets (see Figures 2A and 2B). In these mixtures the E6/reference markers and E2/E6 CNR is 1.

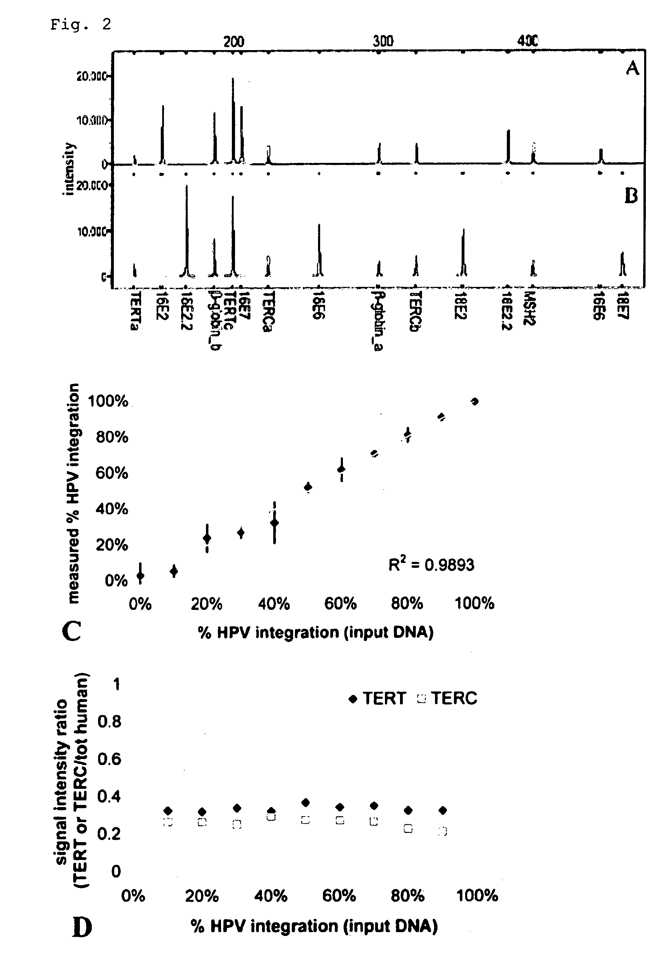

In addition, series with a viral load = 1 and an increasing % of integrated viral copies (E6/reference markers CNR being 1, E2/E6 CNR varying from 1.0 to 0) were prepared. A standard curve to determine the percentage of integrated HPV was prepared for this series by plotting on the x-axis the % of integration (in input DNA) and on the y-axis the calculated % integration determined based on the normalized intensity ratio E2/E6, i.e. the average measured E2/E6 ratio of the reference samples (Figure 2C).

This graph clearly illustrates the linear regression (R2 = 0.9893) between known and measured % HPV integration. Based on the standard deviation of triplicate measurements this standard curve showed that integrated viral copies are recognized when their frequency exceeds 15% of the total number of HPV viral copies. It should be emphasized that in this case a relative copy number differences of 1.18 is detected (E2-100%/E2-85%). Furthermore the standard curve demonstrates that the signal intensity ratio E2/E6 is linear with the copy number for E2, within at least a 10-fold CNR range.

For a proper quantification variable copy numbers of individual targets should not influence the signal intensity ratios for the remaining targets. This was tested for the HPV16/human DNA CNR was 1 series by measuring the signal intensity ratio for either the two TERT or the two TERC targets to all human targets upon varying copy numbers for E2 (figure 2D). The contributions of the TERC and TERT signals to the total human signal intensity were 0.26 ± 0.02% and 0.34 ± 0.02%, respectively. This graph shows that the influence of the varying copy numbers for E2 in the mixtures has a minimal effect on the measured signal intensity ratios. In this series the copy number for the HPV16 E2 targets varied between 0 and 18% of the total copy number for all targets in the PCR reactions. For all other viral probes, as well as all chromosomal targets similar stable correlations were found (data not shown).

### Estimation of viral load

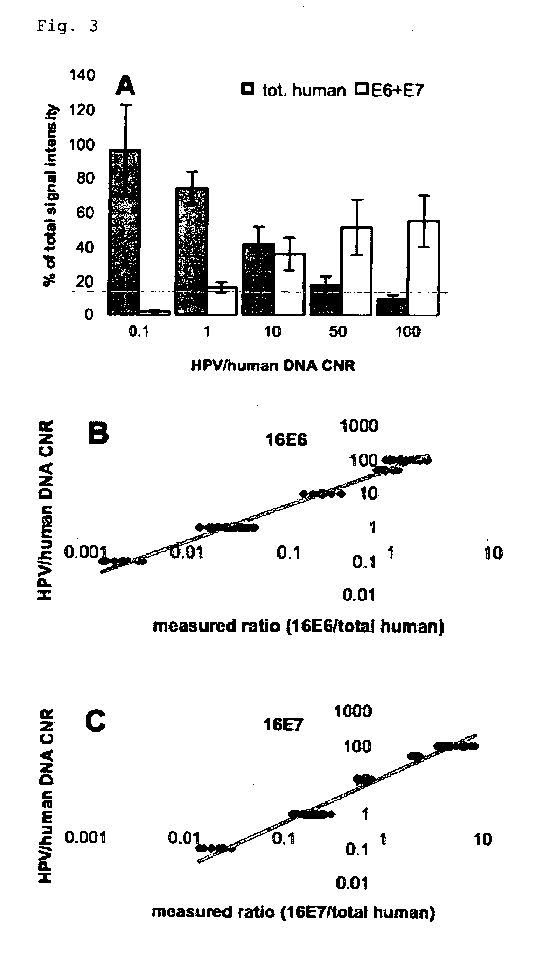

The viral load in clinical samples will vary within a wide range. To examine the effect of a surplus of viral targets over human targets or vice versa, the series with a HPV/human DNA CNR of 100, 50, 10, 1 and 0.1 were analysed and compared. With increasing viral load the signal intensity of the HPV targets increases, although not in a linear manner. For example a 100-fold excess of HPV E6 and E7 results in a three- to four-fold increase in signal intensity. Meanwhile the signal intensity of the human targets decreases, despite the fact that the total amount of human DNA per sample was not altered (Figure 3A). The ratio between HPV and human target signal intensities will therefore provide a better estimate of the viral load. When the measured signal intensities for E6 or E7 were compared to the total of all seven human targets in the five series the linearity increases significantly, but does still not provide a proper estimate of the increase in viral load. To obtain a more realistic estimate of the viral load in a particular sample the correlation was determined between the signal intensity ratios for E6 or E7 and the HPV/human DNA CNR. For this purpose the signal intensity ratios were plotted against the HPV/human DNA CNRs on a double logarithmic scale (see Figures 3B and C for results with HPV16). Based on these graphs for both HPV16 and 18 the mathematical equations as provided in Table 3 were determined. These allow calculation of the viral load (L) for HPV16 and 18 based on the average value of L for E6 and E7.

**Table 3: Equations for estimation of the viral load**

| Target | Equation | R²-vale |
|---|---|---|
| 16 E6 | L = 55.9r₁^{1.1} | 0.9761 |
| 16 E7 | L = 12.5r₁^{1.3} | 0.9699 |
| 18 E6 | L = 16.8r₁^{1.3} | 0.9667 |
| 18 E7 | L = 43.2r₁^{1.4} | 0.9702 |

| | | |
|---|---|---|
| (r₁ = target signal intensity/total human signal intensity; L = viral load) | | |

### Estimation of viral integration

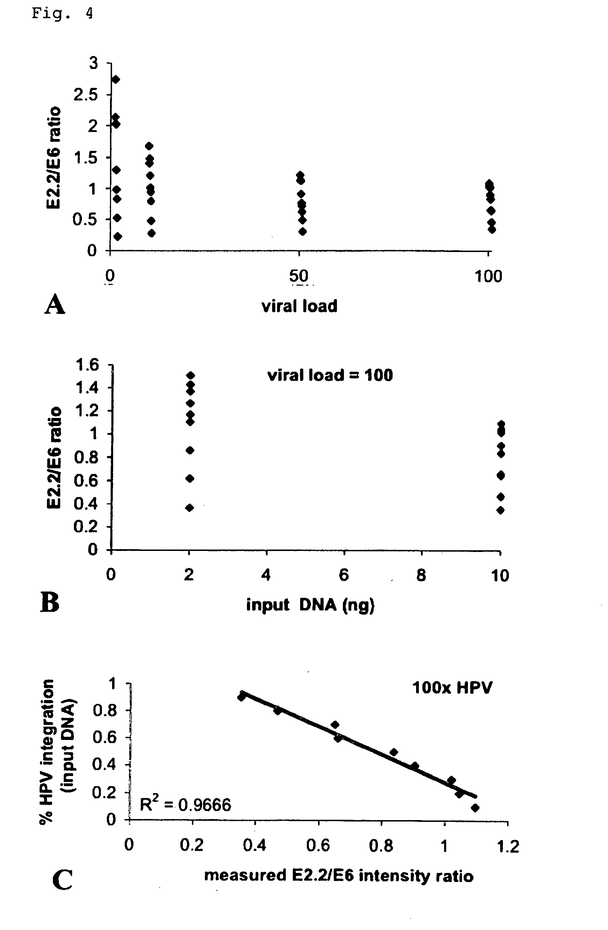

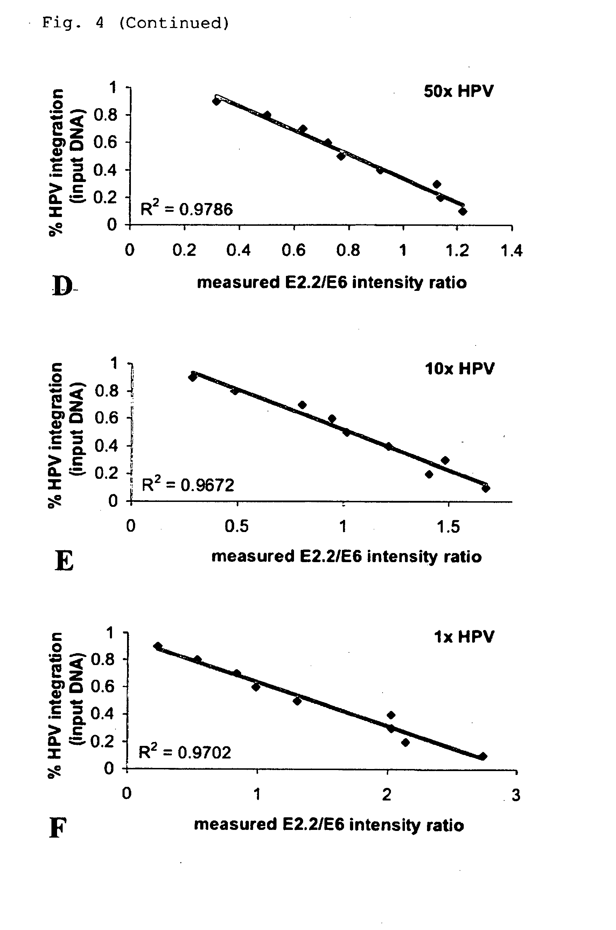

It has been described that viral integration correlates strongly with disease progression. As a consequence of this integration process the E2 gene is (partially) deleted. Quantification of the E2/E6 ratio will therefore allow estimation of viral integration. To test whether or not our MLPA assays allows an accurate distinction of different percentages of viral integration we have used the plasmid samples with varying percentages of integrated viral copies (E2/E6 CNR varying from 0 to 1) and a constant HPV/human DNA CNR of 1. The known percentage of integration was plotted against the measured E2.2/E6 ratio (see Materials and Methods). Figure 4A, shows the dot plots of the E2.2/E6 ratio measured in the mixtures containing 10 to 90% integrated HPV for the four viral load series. The intervals between the subsequent % of viral integration strongly decreased with viral load. However, the measured intensity ratio E2.2/E6 in all series showed to be linear (see Figures 4C-E). In the different series the value E2.1/E6 when 10% and 90% integrated HPV showed to be 3.0, 3.6, 6.0 and 9 for the viral load = 100, 50, 10 and 1, respectively. Only in the viral load = 1 series this difference fitted the expected ratio of 9 based on a linear relation between signal intensity and gene copy number. Mathematical equations as summarized in Table 4, describe the estimation of % of viral integration based on the gene signal intensity ratio for E2.1/E6 and E2.2/E6 when taken into account the viral load of the mixture.

Our observations of smaller intervals (E2/E6 ratios) between subsequent degrees of integration in samples with a high viral load suggest that dilution of these samples would result in an increase of these intervals. This was proven by the 5-fold dilution of the viral load = 100 mixture by which the ratio E2/E6 between 10% and 90% HPV integration increased from 3 to 5 (see Figure 4B). When the CNR HPV/human copies was 0.1 the signal intensity for the HPV targets was too low to reliably analyze the physical status of the virus (data not shown).

**Table 4: Equations for estimation of % viral integration in relation to viral load**

| Viral load | Equation | Equation (Fig 4C-F) |
|---|---|---|
| 100 | y = 100*(-0.52x₁ + 1.19) | y = 100*(-1.02x₂ + 1.30) |
| 50 | y = 100*(-0.52x₁ + 1.20) | y = 100*(-0.87x₂ + 1.21) |
| 10 | y = 100*(-0.30x₁ + 1.02) | y = 100*(-0.58x₂ + 1.10) |
| 1 | y = 100*(-0.16x₁ + 0.93) | y = 100*(-0.32x₂ + 0.95) |

| | | |
|---|---|---|
| (x₁ = signal intensities ratio 16E2.1/16E6; x₂ = signal intensities ratio 16E2.2/16E6; y = % of viral integration) | | |

### MLPA and FISH analysis of HPV cell lines

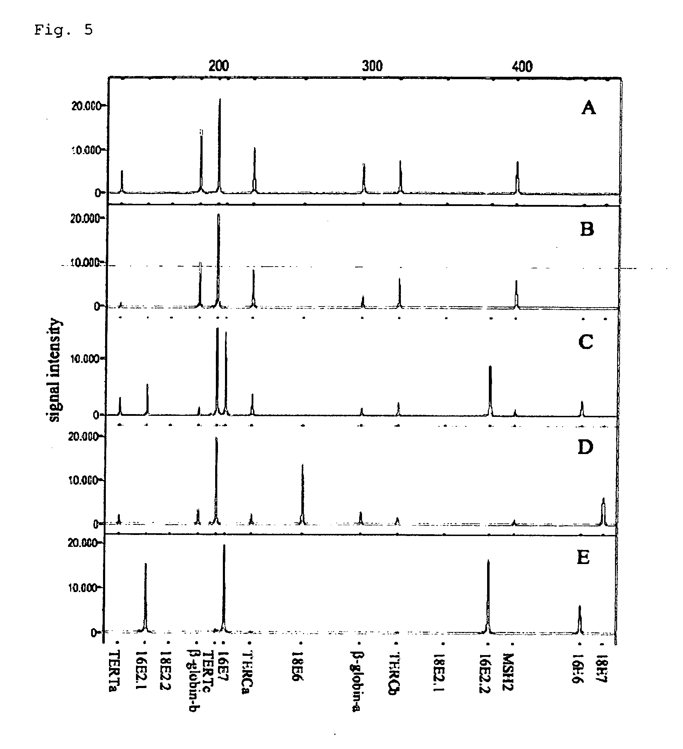

To test the specificity and sensitivity of the assay 7 cell lines were analyzed including four HPV negative cell lines (T47D, MCF7, CaCo-2 and HaCat) and three HPV positive cell lines including SiHa and CaSki containing 1-2 and 60-600 copies of HPV 16, respectively, and HeLa containing 10-50 copies of HPV 18(24). Figure 5 summarizes the capillary electrophoresis peak profiles obtained for the analysis of a 10 ng input of DNA isolated from normal lymphocytes, MCF7, SiHa, HeLa and CaSki. The HPV negative cell lines as well as SiHa and HeLa showed products for all the human targets, while for CaSki only dominant peaks are recognized for the HPV 16 targets.

### Viral Load

For the analysis of the viral load the intensities for E6 and E7 were normalized using the mathematical equations as described in Table 3. By averaging the values for E6 and E7 the viral load in HeLa, SiHa and CaSki was calculated to be 6, 6 and 150 HPV copies per □-globin copy, respectively. A qPCR was performed to measure viral load in the HeLa cell line and to compare these results with the MLPA-assay. A linear relationship was found between the threshold cycle values plotted against the log of the copy number over the entire range of dilutions (data not shown). When the threshold cycle values for the triplicate measurements for HeLa were plotted in this curve an average of approximately 23,000 copies was found. Because we used 10 ng of DNA for this experiment, which should be equal to approximately 1,500 cells, the viral load for HeLa was measured to be 15 HPV 18 copies per cell.

### Viral Integration

For the analysis of the status (integration) of the E2.1 and E2.2 targets in the HPV positive cell lines the intensity ratios E2.1 and E2.2 versus E6 were calculated and the equations as summarized in Table 4 were applied. In HeLa no signals were measured for the 18E2.1 and 18E2.2 targets and calculations showed deletions in HeLa for 18E2.1 and 18E2.2. In SiHa and CaSki signals were measured for 16E2.1 and 16E2.2 but calculations showed 0.3 copies of 16E2.1 per copy of □-globin while 16E2.2 was retained in SiHa and in CaSki both 16E2.1 and 16E2.2 were retained. In SiHa an intensity ratio reduction for E2.1 versus E2.2 (0.3 versus 6 copies) of about 95% was measured suggesting that 5% of the E2.1 targets are retained. Based on these results the cell lines are assessed as having 100% integrated HPV (HeLa), integrated plus episomal HPV (SiHa), and exclusively episomal HPV (CaSki). When the threshold cycle values for the duplicate measurements for SiHa and CaSki were plotted in this curve an average of approximately 77,000 copies and 3,200,000 was found, respectively. Because we used 25 ng of DNA for this experiment, which should be equal to approximately 3,800 cells, the viral load for HeLa was measured to be 20 HPV16 copies per cell for SiHa and 840 HPV 16 copies per cell for CaSki.

### Telomerase related genes

Data for the copy number gains of the telomerase genes as measured by MLPA and FISH are summarized in Table 5. For the analysis of the copy number gain for TERT and TERC the data were normalized for the intensities of both □-globin and MSH2 probes (sum). These intensity ratios were compared to the ratios as measured for these targets in the mixture containing a viral load of 1. When the ratio for the cell line was 1.5x higher compared to the reference sample the cell line was classified as having a copy number gain. The ratios for the cell lines showed a gain for TERT in all cell lines, while an evident gain for TERC was found in CaSki, These data were compared to the FISH analysis for the TERC and TERT gene with the centromere 3 probe as a reference probe for the chromosome ploidy (data not shown). The enumeration of the FISH copy numbers showed that three of the four targets with a copy number gain and one of the two targets without gain in the MLPA assay were confirmed by the FISH copy numbers.

**Table 5: Copy number analysis of HPV containing cell lines by FISH and HPV MLPA-assay**

| | FISH | | | HPV MLPA-assay | |
|---|---|---|---|---|---|
| | copy number (avg ± SD) | | | ratios (avg ± SD) | |
| | TERT | TERC | centromere 3 | TERT | TERC |
| CaSki | 6.1 ± 1.0 | 5.0 ± 1.0 | 3.9 ± 0.7 | 6.0 ± 1.0 | 3.1 ± 0.5 |
| SiHa | 6.3 ± 1.4 | 2.9 ± 0.6 | 2.4 ± 0.7 | 2.3 ± 0.2 | 1,6 ± 0.2 |
| HeLa | 4.5 ± 0.7 | 3.8 ± 0.6 | 3.8 ± 0.7 | 1.9 ± 0.1 | 0.7 ± 0.1 |

### Application of the MLPA assay in clinical samples

To test the performance of the HPV MLPA assay in different types of clinical samples, we selected a limited series of fresh-frozen and paraffin-embedded tissue samples and cytological specimens. In these cases viral type, load and integration and gain of TERC were determined in duplicate with the MLPA assay and validated with qPCR and FISH-(*infra*). In these 3 formats, matching results were found between the assays with respect to HPV typing and load. Also, other independent HPV assays, including GP5□/6□ PCR and p16 immunostaining analysis showed matching results (data not shown). Furthermore, the MLPA assay quantified viral loads ranging from 0.1 to 532 viral copies per diploid genome, with small differences in viral load between duplicate measurements. Bottom line, in a number of clinical samples ranging from normal to carcinoma, the MLPA assay was able to discriminate between high- and low-grade lesions based on the presence of viral integration and/or a high-viral load, as verified by independent assays. As will be discussed in more detail hereinafter, we can conclude that the new multiparameter HPV MLPA assay described herein allows a reliable detection and quantification of HPV16 and 18. Furthermore, the procedure enables the detection of viral integration in samples with different viral loads and can also be combined with analysis of genomic instability based on gain of telomerase-related genes. Application of the assay in clinical samples will pave the way to improve risk assessment for patients suspected for HPV infection.

### HPV-typing

In order for the MLPA-assay to be useful in cervical screening the sensitivity in HPV-typing has to be high. As summarized in Table 3, 52 of the 67 patient samples were found to be positive for HPV16, including 4 of the 7 (57%) normal samples, 14 of the 20 (70%) normal samples adjacent to a lesion, 7 of the 10 (70%) CIN1/condyloma samples, 12 of the 13 (92%) CIN2/3 samples and 15 of the 17 (88%) carcinoma samples. Furthermore, one CIN2/3 and one carcinoma sample were found to be positive for HPV18.

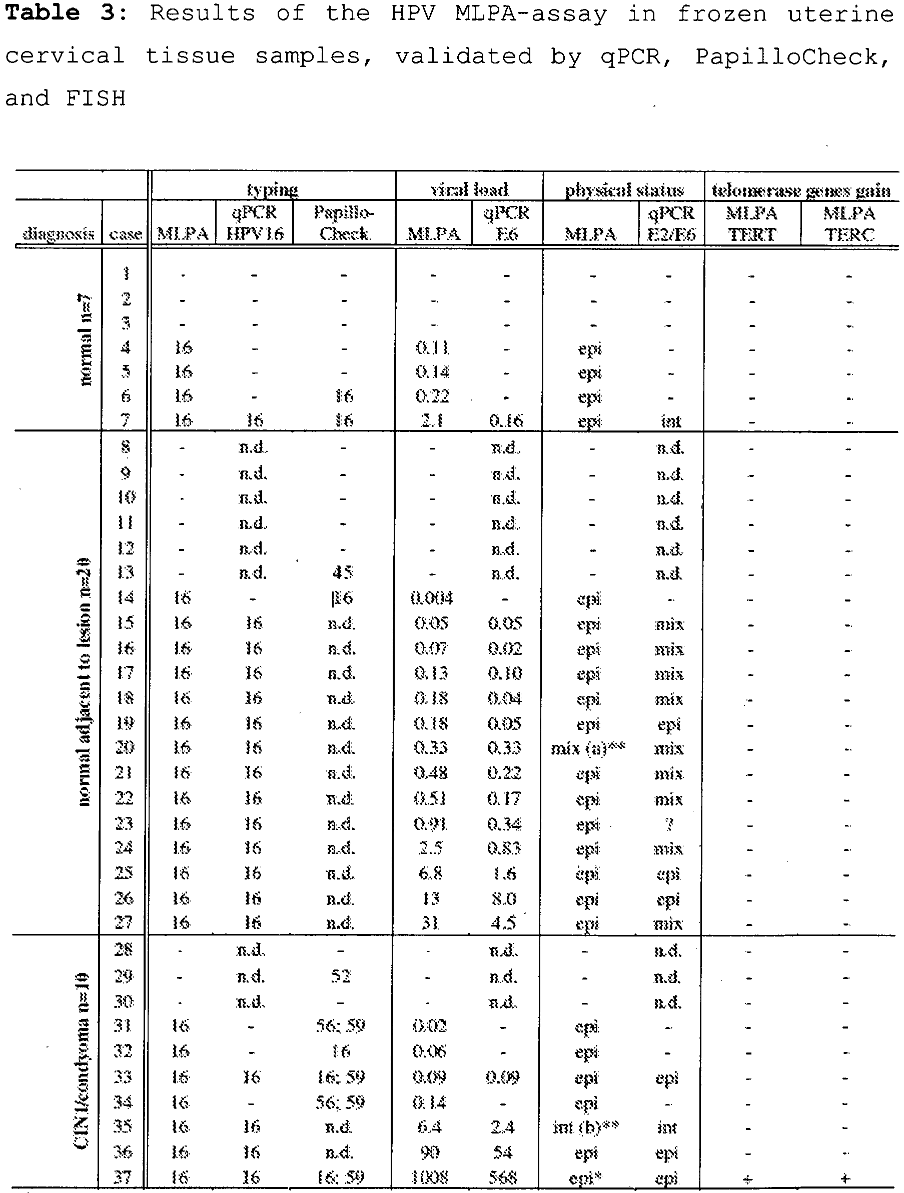

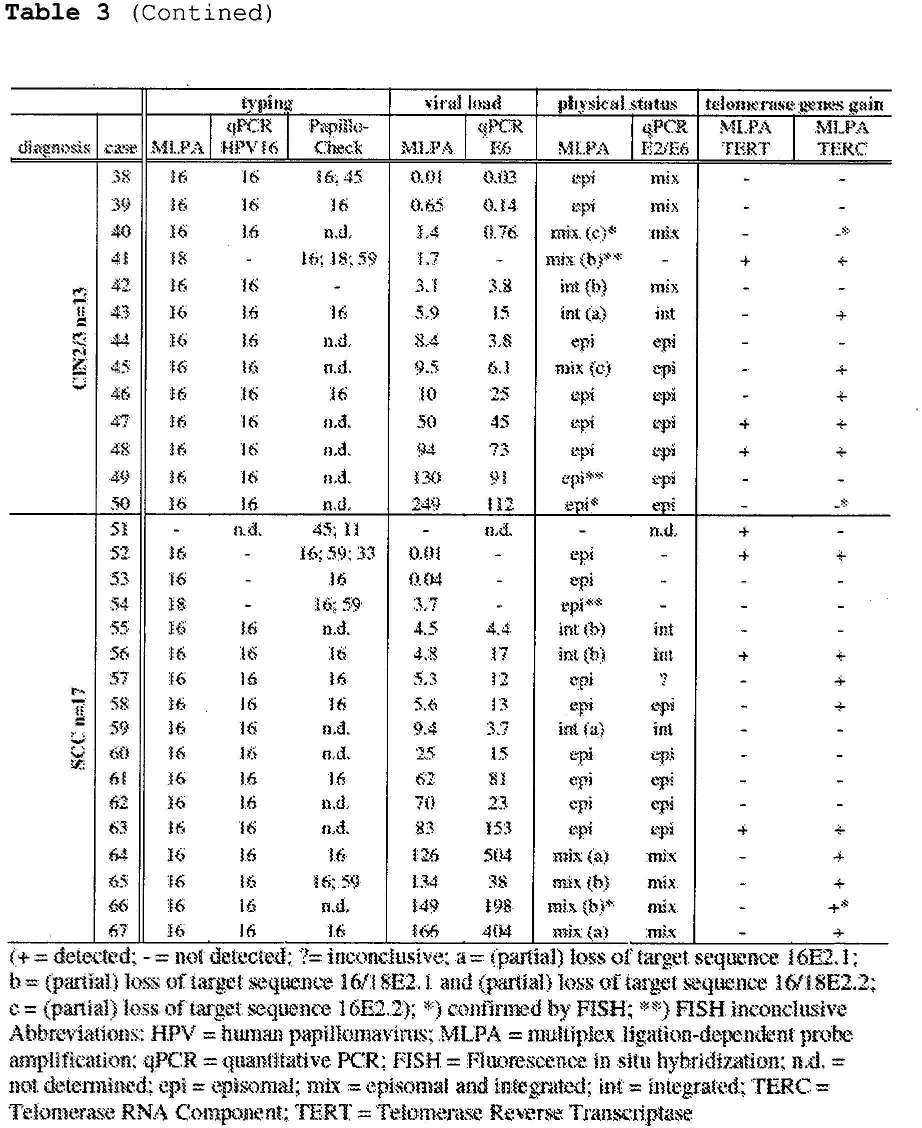

To determine the validity of the MLPA results all samples that were positive for HPV16 using the MPLA-assay were validated with qPCR for HPV16. Of the 52 samples that were determined to be HPV16 positive using the MLPA-assay, 43 were confirmed by qPCR (Table 4) and 9 samples, all with a viral load of less than 1 copy per 4 cells, were negative.

**Table 4: MLPA typing versus qPCR**

| MLPA | qPCR | | |
|---|---|---|---|
| | total | 16+ | 16- |
| 16+ | 52 | 43 | 9 |
| 18+ | 2 | 0 | 2 |

Furthermore, 24 randomly selected HPV16 positive samples were validated using the PapilloCheck. Nineteen of the 24 (79%) HPV16 positive samples were confirmed, while 4 of the 5 samples in which no HPV16 was detected with the PapilloCheck, were determined to have a viral load of less than 1 copy per 5 cells, and the fifth sample had a viral load of 3 copies per cell. In two of these negative samples a double infection with HPV56 and 59 was detected and the other three samples were negative for all HPV types detected by the PapilloCheck (Table 5).

**Table 5: MLPA typing versus PapilloCheck**

| MLPA | PapilloCheck | | | | | |
|---|---|---|---|---|---|---|
| | total | 16+ | 18+ | 16+ 18+ | others | negative |
| 16+ | 24 | 19 | - | - | 2 | 3 |
| 18+ | 2 | 1 | - | 1 | - | - |
| 16- 18- | 13 | - | - | - | 3 | 10 |

| | | | | | | |
|---|---|---|---|---|---|---|
| - = not detected | | | | | | |

All 15 samples that were determined to be HPV16 negative by the MLPA-assay were validated using the PapilloCheck, which was also used to identify infections with other HPV types. As summarized in Table 3 and 5 these samples included the two HPV18 positive samples, which the PapilloCheck determined to be positive for HPV16 or positive for HPV16 and 18. No HPV16 could be detected by qPCR in these two samples (cases 41 and 54 in Table 3; Table 4). There were 3 samples that were positive for one or more of the other HPV types. This concerned an HPV45 infection in a normal sample which was adjacent to a lesion, an HPV52 infection in a CIN1/condyloma sample and a co-infection with HPV11 and 45 in a carcinoma sample. The 10 remaining samples were negative for all HPV types detected by the PapilloCheck.

### Viral Load

In addition to a high sensitivity in HPV-typing, for the MLPA-assay to be useful in cervical screening, it must have a predictive value in the viral load, as the latter is associated with progression to cancer.

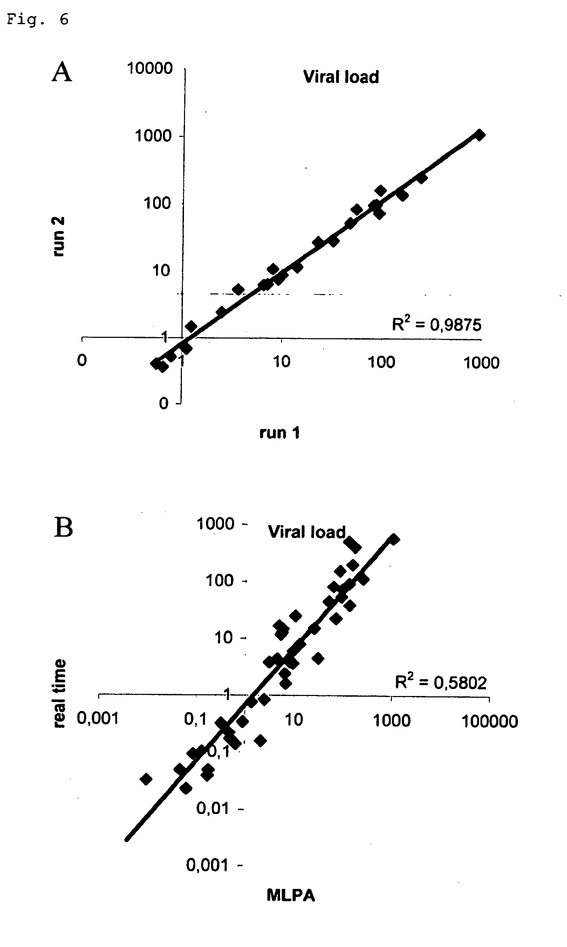

Overall the viral load detected by the MLPA-assay ranged from less than 1 copy per 10 cells to approximately 1000 copies per cell. As depicted in Figure 6A the reproducibility of the MLPA procedure for viral load detection between the two duplicated analyses, starting from the same DNA sample, is very high. A maximum viral load of 2 copies per cell was detected for the normal samples, 31 copies per cell for the normal samples adjacent to a lesion, 1008 copies per cell for the CIN1/condyloma samples, 249 copies per cell for the CIN2/3 samples, and 166 copies per cell for the carcinoma samples.

To validate the viral load as determined by the MLPA-assay, all HPV16 positive simples were analysed by qPCR for HPV16 E6. Overall the viral load detected by qPCR ranged from less than 1 copy per 10 cells to approximately 568 copies per cell, with a viral load of 0.16 copies per cell for the normal sample, and a maximum viral load of 8 copies per cell for the normal samples adjacent to a lesion, 568 copies per cell for the CIN1/condyloma samples, 112 copies per cell for the CIN2/3 samples, and 504 copies per cell for the carcinoma samples. When comparing the viral loads as determined by the MLPA-assay or qPCR for the individual cases it became evident that there is a good correlation in that both procedures detect a similar range of HPV copies per cell (Figure 6B) for the patient samples.

### Viral integration

A further parameter associated with progresion to cancer is the physical status of the virs in the host. For said reason the MLPA-assay was further validated on its performance in determining the physical status of the virus in the tissue samples.

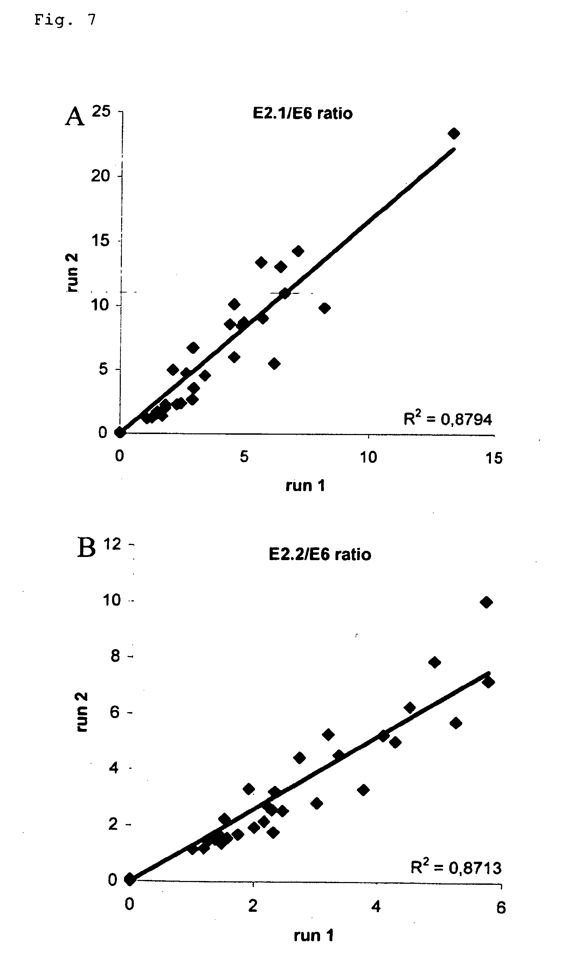

Viral integration into the host genome is described to be associated with progression to cancer. As a consequence of this integration (part of) the HPV E2 gene is almost always deleted, while the HPV E6 gene is retained. Quantification of the E2/E6 ratio is therefore used for the estimation of viral integration. The reproducibility of both the E2.1/E6 and the E2.2/E6 signal intensity ratio is depicted in figure 7A and B and found to be high.

Integrated HPV was detected in 1 of the 16 HPV16 positive normal samples adjacent to a lesion, 1 of the 7 HPV16 positive CIN1/condyloma samples, 4 of the 12 HPV16 positive CIN2/3 samples as well as the HPV18 positive CIN2/3 sample and 7 of the 15 HPV16 positive carcinoma samples.

For the validation of the physical status as determined by MLPA-assay E2/E6 duplex qPCR was used. Thirteen of the 14 samples that were determined to contain integrated HPV by the MLPA-assay, were confirmed by qPCR. One of these samples (case 42) was determined to contain predominantly integrated HPV by the MLPA-assay whereas qPCR determined the sample to contain both integrated and episomal HPV. In addition, 17 of the 39 samples that were determined to be episomal by MLPA were confirmed by qPCR. Nine of these 39 samples could not be confirmed because no HPV16 was detected by qPCR and in 2 of these 39 samples qPCR was inconclusive because of inconsistency between duplicates. Strikingly, in the remaining 11 samples in which the MLPA-assay detected episomal HPV and which comprised 9 normal samples, of which 8 adjacent to a lesion, and 2 CIN2/3 samples (summarized in Table 6) the qPCR indicated the presence of integrated HPV.

**Table 6: Comparison between MLPA and qPCR for physical status analysis.**

| **MLPA** | **qPCR** | | | | | |
|---|---|---|---|---|---|---|
| | total | epi | mix | Int. | ? | - |
| epi | 39 | 17 | 10 | 1 | 2 | 9 |
| mix 21 | 3 | - | 3 | - | - | - |
| mix 2.2 | 2 | 1 | 1 | - | - | - |
| mix 2.1 and 2.2 | 2 | - | 2 | - | - | - |
| int 2.1 | 4 | - | - | 2 | - | - |
| int 2.1 and 2.2 | 4 | - | 1 | 3 | - | - |

| | | | | | | |
|---|---|---|---|---|---|---|
| epi = episomal siks = episomal and integrated int = integrated; 2.1 = (partial) loss of targetsequence 1.6132.1: 2.2 = (partial) less of targetsequence 16E2.2; ?= inconclusive: - = next detected | | | | | | |

Of the 10 samples that were analysed for the physical status of HPV16 and HPV18 by FISH episomal HPV16 was confirmed in three cases (cases 27, 37 and 50 in Table 3) and integrated HPV in two cases (cases 40, and 66 in Table 3). In the remaining 3 HPV16 samples (cases 20, 35 and 49) FISH analysis was inconclusive, which was also the case for the 2 samples that were analysed for HPV18 (cases 41 and 54). All these latter 5 cases had a relatively low viral load.

### Telomerase genes

Gain for the telomerase related genes TERT and TERC is described to be associated with progression to cancer, and was therefore included in our HPV MLPA-assay. Gain for TERT was detected in 8 samples, i.e. 1 CIN1, 3 CIN2/3, and 4 carcinoma samples. Gain for TERC was detected in 16 samples, i.e. 1 CIN1, 6 CIN2/3, and 9 carcinoma samples. Seven of the 8 cases with gain for TERT overlapped with the TERC gain cases. Only in case 51 TERT gain was seen to be independent of gain for TERC. The FISH analyses for TERC performed on tissue sections from three patients (cases 40, 50, and 66 in Table 3) gave confirmatory results.

### Sensitivity and specificity of the MLPA-assay

To quantify the diagnostic capacity of the MLPA-assay in the tissue samples, sensitivity and specificity were calculated for each of the 4 parameters, i.e. HPV type 16/18, viral load, viral integration and gain of the telomerase related genes. For this purpose the ability of the assay to distinguish high grade CIN2/3 and carcinoma lesions on the one hand, from normal samples, low grade CIN1 and condyloma lesions on the other hand, was determined. For the presence of HPV type 16 and 18 the sensitivity is calculated to be 97% but the specificity to be only 32%. For a viral load of more than 25 copies per cell the sensitivity and specificity are calculated to be 40% and 92%, respectively. For viral integration this is 40% and 95%, for gain of TERT 23% and 97%, and for gain of TERC 50% and 97%, respectively (Table 7).

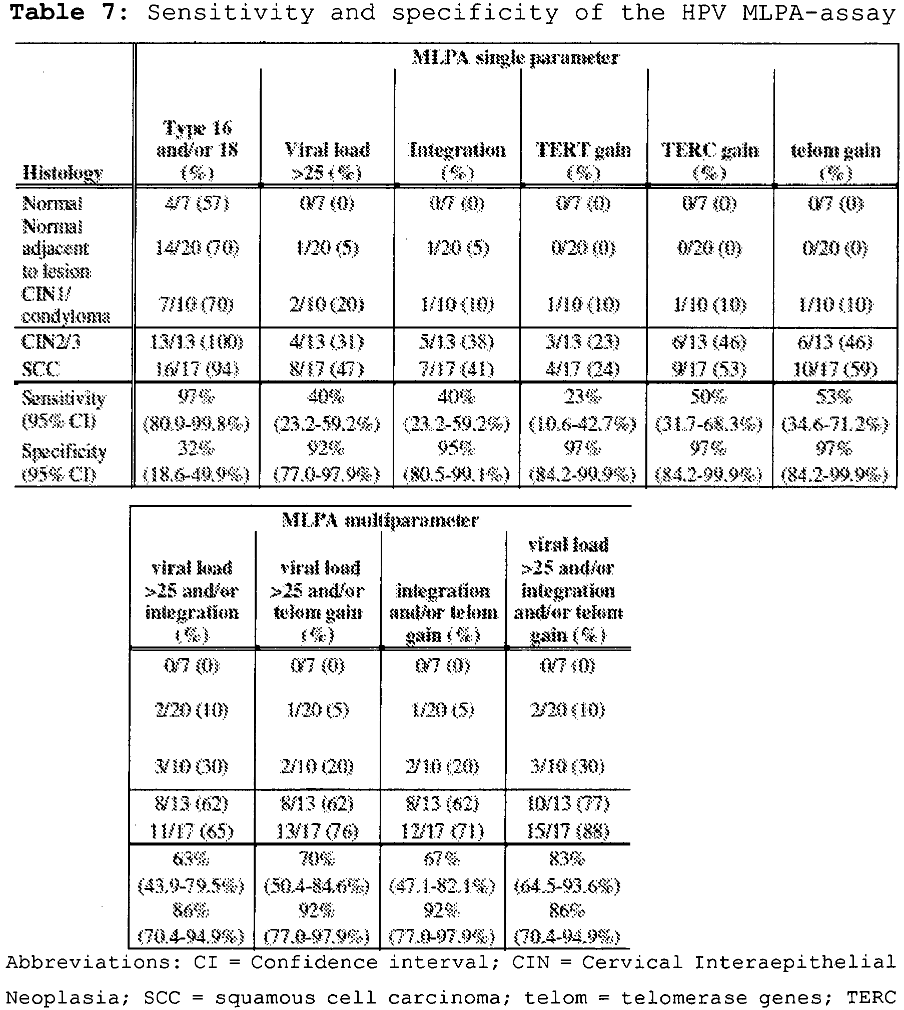

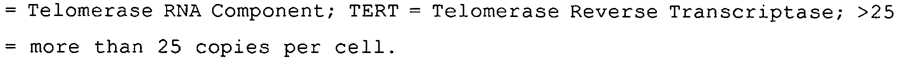

By combining 2 parameters the sensitivity can be increased up to 63-70%, with a small reduction in specificity to 86-92%. When all parameters are combined the sensitivity increases up to 83%, while retaining a specificity of 86%.

### MLPA-assay in cytology

Cytological samples of 20 cases, ranging from normal to dyskaryotic and for which the presence of HPV was known based on GP5+/6+ testing 22 and/or the PapilloCheck, were analyzed by the MLPA-assay and partly verified by quantitative PCR. The MLPA-assay determined 10 samples to be negative for HPV16 or 18. Five of these samples were found to be positive for 1 or more other HPV types using GP5+/6+ testing and the PapilloCheck and the other 5 samples were indeed negative for all HPV types detected by both assays. For the 10 positive cases, both the typing and viral load were were confirmed by GP5+/6+ testing, the PapilloCheck and qPCR (data not shown). Also the presence of integrated HPV, which was detected in the MLPA assay in 2 HPV18 en 2 HPV16 cases, was partially confirmed by qPCR. Conformity in gain for TERT and TERC was less than expected, but this could be due to the fact that the fraction of (pre)malignant cells is to small to pass the treshold for gain of the telomerase associated genes. Notwithstanding, in 2 cases gain for TERT and TERC was detected. This convincingly shows that despite the fact that cytological samples contain a large amount of normal cells, the MLPA-assay is able to detect gain for TERT and TERC. It accordingly demonstrates that even in cytological specimens the MLPA assay allows in a single test to determine HPV type, load and integration (physical status).

### Discussion

Our study showed that our multiparameter MLPA assay based on the 15 selected targets can reliably assess HPV 16/18, load and integration status of the virus together with copy number gain of telomerase genes (TERT and TERC). The MLPA HPV E2, E6 and E7 probes demonstrated to be type specific, allowed measurements of viral loads of 0.5 to 100 (CNR HPV/human copies) and a detection sensitivity of viral integration up to 10-20%. To enable a reliable calculation of the viral load and the fraction of integrated versus episomal virus based on the capillary electrophoresis peak profile the correlation between copy number and signal intensity is best determined. Our observation that an input of 10 ng of human DNA provided an internal reference for normalization resulted in equations that allowed a reliable calculation of viral load and % integration. Copy number gains (2-3 times) of telomerase associated genes were determined by normalization for the signal intensity ratios with the reference genes □-globin and MSH2. The impact of the viral load to measure copy number gain of these telomerase genes was negligible.

### HPV MLPA-assay versus conventional MLPA assay

The conventional MLPA-assay is a powerful assay to differentiate between gene CNR of 0.5 (monosomy), 1.5 (trisomy), >2 (amplifications) or 0 (homozygous deletion). For a monosomy or trisomy the signal intensities for the gene will show a 50% decreased or increased, respectively. In the HPV MLPA-assay we analyzed two ranges of CNRs for HPV targets e.g. a small range for integration to discriminate between 10 and 9 copies E2 to measure 10% integration and a wide range for viral load to discriminate between 1 to 100 copies E6 in a background of 1 copy human DNA. The HPV MLPA-assay showed to be quantitative over several magnitudes of copy number differences for the different targets where E2/E6 was used to measure integration (range 1-0.1) and E6 for load. Although the conventional MLPA assays are normally used to measure limited copy number differences, the copy number detection sensitivity of the HPV MLPA-assay surprisingly showed that this sensitivity is some magnitudes higher. The mathematical corrections as shown in the different tables are simple reflections for the competition between the different targets for amplification. If the HPV load is high (>50) peaks for human targets are strongly reduced due to competition during the PCR. The same accounts for a low viral load (<0.1) in these cases human signals are dominating. Notwithstanding the HPV-MLPA assay is able to give a reliable assessment of viral load, viral integration and human disease progression markers in a single assay. In the HPV-MLPA assay an optional pre-amplification step is incorporated, this is done to increase sensitivity and to facilitate detection of very small amounts of HPV copies. For the majority of clinical cases the HPV viral load is found to be at least one copy per cell (26), through the incorporation of this pre-amplification only a small amount of human DNA (10 ng, 3000 copies) is needed for analysis. In case the viral load is too low the reaction could be repeated with a 5- or 10-fold increase of input DNA.

### MLPA versus real time PCR

In the HPV-MLPA assay the ratio between E2 and E6 or E7 is used to determine the integration status of the virus. Although it is new to use an MLPA-assay for this purpose, the use of this ratio is not. In real-time PCR experiments this ratio is frequently used to reliably measure viral integration (9, 26, 28). The advantage of the HPV MLPA assay compared to these real-time PCR assays is the possibility to simultaneously analyze and quantify additional altered sequences (see below). Furthermore our HPV MLPA-assay showed that it is possible to distinguish a larger group of mixed cases where the real-time PCR would label part of these as episomal (28). Furthermore the copy number differences for 30-40 targets can be measured in a single reaction.

### MLPA versus APOT, DIPS and FISH

DIPS, APOT identified integrated HPV16 and/or 18 in all analyzed fresh frozen samples, while the MLPA assay detected viral integration in the paraffin embedded tissue of 12 of these 13 cervical carcinomas cases (data not shown). In the remaining case episomal HPV was identified, most probably due to absence of deletion within the MLPA E2 target sequences. FISH confirmed integration in 8 out of 13 tumors and was inconclusive in the remaining cases due to low viral loads. In conclusion we can state that the HPV-MLPA assay is a reliable alternative to the other (APOT, DIPS and FISH) assays for detection of HPV-integration in paraffin embedded tissue material.

### Selection of E2 probes

In our study two probes directed against different positions in the E2 gene were selected to determine viral integration. For HPV 16 these probes were located within the two regions that were described to be most often deleted (1, 34), for HPV 18 this detailed information is not available and the two probes were randomly selected. In most cases E2 expression is affected upon integration of viral DNA into the human genome as a result of a deletion of E2. In addition deletions of the E1 gene are also described to disrupt E2 expression (1, 34, 37). Furthermore deletions in these genes are not limited to special locations and can occur all over the gene (1, 34). By using two probes for the detection of integration in stead of one the amount of integrated cases that can be detected will be increased but still it is possible that some cases of integration will be missed. The probability to detect viral integration can be increased by adding additional probes dispersed over the E1 and E2 genes. Analysis of the cell lines showed that the selected probes enabled detection of viral integration in case of HeLa (HPV 18). The CaSki cell line contains a large number of integrated HPV 16 genomes. However, the E2 targets are retained in these integrated viral genomes. Therefore, the integrated viral HPV 16 genomes are not identified as such. For the cell line SiHa a deletion of E2.1 (position 3460-3512) and retention of E2.2 based on the published data (24) was expected. We clearly detected a reduction of the copy numbers for E2.1, up to 70%, as compared to E2.2 however E2.1 was still present. Heterogeneity of the SiHa cell line for the E2.1 region could explain these findings. This is supported by our previous observation that nuclei of SiHa cells show one or two HPV signals after HPV 16 FISH analysis and by disagreements in sequence analysis of SiHa (11, 24).

### Calculation of viral load and % integration

To enable a reliable calculation of viral load and the fraction of integrated versus episomal virus based on the capillary electrophoresis peak profile, the plasmid model system was applied to study the correlation between copy number and signal intensity obtained in this assay. For the determination of viral load the competitive response of the signals in the multiplex PCR in the MLPA assay which reflects a relative abundance of HPV and gene targets was taken into account. Mathematical equations showed to enable description of the viral load as function of the target signal intensity (E6 or E7) and human signal intensities. Furthermore, the % HPV integration showed to be viral load dependent. The most important observation was that smaller intervals (E2/E6 ratios) between subsequent degrees of integration in mixtures are measured when the viral load increases. This reduction inevitably would lead to misclassification and overestimation of the degree of integration. Only in the viral load = 1 series no mathematical correction is needed. The ratio E2/E6 after normalization (measured ratio E2/E6 = 1 for 100% episomal virus) directly indicates the fraction op episomal virus. A consequence of this interval constrain is the reliability to detect minimal amounts of integrated HPV. In a single measurement 10-20% integration could be recognized in case of a low viral load, with increasing load this % gradually increases. In the latter case a simple dilution of the sample and re-analysis of the sample will strongly improve the accuracy up to 10-20%..

Utilization of the equations for the analysis of load in the HPV containing cell lines SiHa, Hela and CaSki showed that they fall within the range reported in the literature. If we take the chromosome ploidy (3-4) into account the HPV-MLPA-assay showed 18 to 24 copies per cellular genome for HeLa which is close to the detected copies by qPCR(22).

### Gain of telomerase genes

The ratios for the cell lines showed a gain for TERT in all cell lines, and gain for TERC in CaSki. This gain was relative to the copy numbers for □-globin and MSH2. These data were comparable with the FISH data, where centromere 3 was used as a copy number reference. The MLPA data confirmed the high resolution array CGH data showing gain of TERT in all cell lines, TERC gain in CaSki while TERC was not gained in SiHa and HeLa (20).

### HPV MLPA-assay versus conventional HPV assays

As is evident from the aforementioned results, For typing of HPV16 and 18 all the samples that were determined to be negative by the MLPA-assay were confirmed by qPCR and/or the PapilloCheck. This was also the case for 48 of the 52 samples that were found to be HPV16 positive. The discrepant samples were characterized by a low viral load, which implies that the MLPA-assay is at least as sensitive as qPCR and the PapilloCheck in the detection of these HPV types. This is particularly surprising since for these two latter assays higher amounts of DNA were used as compared to the MLPA-assay. The more in that for the qPCR even a larger number of cycles, 50 instead of 40, was used when compared to the standard HPV16 qPCR test.

Also the risk of false positive results is, unlikely in the MLPA-assay because of the use of 4 probes per HPV type. For example, HPV16 infection is only scored as being positive when a product is detected for both the E6 and E7 probe, while in a large group of cases also products will be detected for one or both of the E2 probes, providing additional proof for the reliability of the outcome. Furthermore, the simples in our study were randomly selected without knowledge of HPV status, and as described above mostly confirmed by qPCR or the PapilloCheck providing additional arguments against false positive MLPA-results.

The presence of HPV in the normal samples, either without a lesion or adjacent to a premalignancy, indicates that the sole detection of HPV is not a very specific prognosticator for the presence of a high grade lesion. An additional parameter in cancer prognosis is viral load. In the literature the cut-off value for viral load used to identify with high specificity women with prevalent high grade lesions is described to be between 22 and 35 copies per cell. Therefore, the cut-off value used in this study was set at 25 viral copies per cell. Based on this value 40 of the 43 samples were identically classified using either the MLPA-assay or qPCR. In this study, the viral load as detected using the MLPA-assay was generally somewhat higher than the viral load as detected using qPCR, which might be a motivation to use a higher cut-off value for the MLPA-assay instead.

### In conclusion

The new multiparameter HPV MLPA-assay showed that copy number differences from 1.18 up to several magnitudes can be reliably detected and quantified.

With respect to the discriminating power of the MLPA-assay in patient samples a sensitivity and specificity of 63% and 86%, respectively, were obtained by combining the three HPV parameters. Inclusion of the results for the telomerase related genes in these analyses leads to an increase of the sensitivity to 83%, while retaining the specificity of 86%.

This makes the assay a simple one tube procedure that allows the detection of viral integration in samples with different viral loads. The limited number of probes included in the assay so far demonstrates the applicability of the assay and shows that mathematical corrections are needed to reduce overestimation of viral integration. Extension of the number of viral and human probes will improve the detection sensitivity of viral integration and detection of genomic instability. Application of the assay in clinical samples opens the way to improve risk assessment for patients suspected for HPV infection.

In conclusion, we showed that the HPV MLPA-assay is capable of distinguishing high grade from low grade cervical lesions, both with a high sensitivity and high specificity and constitutes therefore, a valuable screening tool for cervical (pre)neoplasia.

### References

1. Arias-Pulido, H., C. L. Peyton, N. E. Joste, H. Vargas, and C. M. Wheeler. 2006. Human papillomavirus type 16 integration in cervical carcinoma in situ and in invasive cervical cancer. J Clin Microbiol 44:1755-62.
2. Badaracco, G., and A. Venuti. 2005. Physical status of HPV types 16 and 18 in topographically different areas of genital tumours and in paired tumour-free mucosa. Int J Oncol 27:161-7.
3. Badaracco, G., A. Venuti, A. Sedati, and M. L. Marcante. 2002. HPV16 and HPV18 in genital tumors: Significantly different levels of viral integration and correlation to tumor invasiveness. J Med Virol 67:574-82.
4. Bosch, F. X., A. Lorincz, N. Munoz, C. J. Meijer, and K. V. Shah. 2002. The causal relation between human papillomavirus and cervical cancer. J Clin Pathol 55:244-65.
5. Boshart, M., L. Gissmann, H. Ikenberg, A. Kleinheinz, W. Scheurlen, and H. zur Hausen. 1984. A new type of papillomavirus DNA, its presence in genital cancer biopsies and in cell lines derived from cervical cancer. Embo J 3:1151-7.
6. Chen, C. M., M. P. Shyu, L. C. Au, H. W. Chu, W. T. Cheng, and K. B. Choo. 1994. Analysis of deletion of the integrated human papillomavirus 16 sequence in cervical cancer: a rapid multiplex polymerase chain reaction approach. J Med Virol 44:206-11.
7. Cheung, J. L., K. W. Lo, T. H. Cheung, J. W. Tang, and P. K. Chan. 2006. Viral load, E2 gene disruption status, and lineage of human papillomavirus type 16 infection in cervical neoplasia. J Infect Dis 194:1706-12.
8. Clifford, G., S. Franceschi, M. Diaz, N. Munoz, and L. L. Villa. 2006. Chapter 3: HPV type-distribution in women with and without cervical neoplastic diseases. Vaccine 24 Suppl 3:S26-34.
9. Cricca, M., A. M. Morselli-Labate, S. Venturoli, S. Ambretti, G. A. Gentilomi, G. Gallinella, S. Costa, M. Musiani, and M. Zerbini. 2007. Viral DNA load, physical status and E2/E6 ratio as markers to grade HPV16 positive women for high-grade cervical lesions. Gynecol Oncol 106:549-57.
10. Dalstein, V., D. Riethmuller, J. L. Pretet, K. Le Bail Carval, J. L. Sautiere, J. P. Carbillet, B. Kantelip, J. P. Schaal, and C. Mougin. 2003. Persistence and load of high-risk HPV are predictors for development of high-grade cervical lesions: a longitudinal French cohort study. Int J Cancer 106:396-403.
11. Hafkamp, H. C., E. J. Speel, A. Haesevoets, F. J. Bot, W. N. Dinjens, F. C. Ramaekers, A. H. Hopman, and J. J. Manni. 2003. A subset of head and neck squamous cell carcinomas exhibits integration of HPV 16/18 DNA and overexpression of p16INK4A and p53 in the absence of mutations in p53 exons 5-8. Int J Cancer 107:394-400.
12. Heselmeyer, K., E. Schrock, S. du Manoir, H. Blegen, K. Shah, R. Steinbeck, G. Auer, and T. Ried. 1996. Gain of chromosome 3q defines the transition from severe dysplasia to invasive carcinoma of the uterine cervix. Proc Natl Acad Sci U S A 93:479-84.
13. Ho, G. Y., R. Bierman, L. Beardsley, C. J. Chang, and R. D. Burk. 1998. Natural history of cervicovaginal papillomavirus infection in young women. N Engl J Med 338:423-8.
14. Hopman, A. H., F. Smedts, W. Dignef, M. Ummelen, G. Sonke, M. Mravunac, G. P. Vooijs, E. J. Speel, and F. C. Ramaekers. 2004. Transition of high-grade cervical intraepithelial neoplasia to micro-invasive carcinoma is characterized by integration of HPV 16/18 and numerical chromosome abnormalities. J Pathol 202:23-33.
15. Hopman, A. H., W. Theelen, P. P. Hommelberg, M. A. Kamps, C. S. Herrington, L. E. Morrison, E. J. Speel, F. Smedts, and F. C. Ramaekers. 2006. Genomic integration of oncogenic HPV and gain of the human telomerase gene TERC at 3q26 are strongly associated events in the progression of uterine cervical dysplasia to invasive cancer. J Pathol 210:412-9.
16. Huang, F. Y., Y. K. Kwok, E. T. Lau, M. H. Tang, T. Y. Ng, and H. Y. Ngan. 2005. Genetic abnormalities and HPV status in cervical and vulvar squamous cell carcinomas. Cancer Genet Cytogenet 157:42-8.
17. Kalantari, M., F. Karlsen, G. Kristensen, R. Holm, B. Hagmar, and B. Johansson. 1998. Disruption of the E1 and E2 reading frames of HPV 16 in cervical carcinoma is associated with poor prognosis. Int J Gynecol Pathol 17:146-53.
18. Klaes, R., S. M. Woerner, R. Ridder, N. Wentzensen, M. Duerst, A. Schneider, B. Lotz, P. Melsheimer, and M. von Knebel Doeberitz. 1999. Detection of high-risk cervical intraepithelial neoplasia and cervical cancer by amplification of transcripts derived from integrated papillomavirus oncogenes. Cancer Res 59:6132-6.
19. Klussmann, J. P., S. J. Weissenborn, U. Wieland, V. Dries, H. E. Eckel, H. J. Pfister, and P. G. Fuchs. 2003. Human papillomavirus-positive tonsillar carcinomas: a different tumor entity? Med Microbiol Immunol 192:129-32.
20. Lockwood, W. W., B. P. Coe, A. C. Williams, C. MacAulay, and W. L. Lam. 2007. Whole genome tiling path array CGH analysis of segmental copy number alterations in cervical cancer cell lines. Int J Cancer 120:436-43.
21. Luft, F., R. Klaes, M. Nees, M. Durst, V. Heilmann, P. Melsheimer, and M. von Knebel Doeberitz. 2001. Detection of integrated papillomavirus sequences by ligation-mediated PCR (DIPS-PCR) and molecular characterization in cervical cancer cells. Int J Cancer 92:9-17.
22. Macville, M., E. Schrock, H. Padilla-Nash, C. Keck, B. M. Ghadimi, D. Zimonjic, N. Popescu, and T. Ried. 1999. Comprehensive and definitive molecular cytogenetic characterization of HeLa cells by spectral karyotyping. Cancer Res 59:141-50.
23. Matthews, C. P., K. A. Shera, and J. K. McDougall. 2000. Genomic changes and HPV type in cervical carcinoma. Proc Soc Exp Biol Med 223:316-21.
24. Meissner, J. D. 1999. Nucleotide sequences and further characterization of human papillomavirus DNA present in the CaSki, SiHa and HeLa cervical carcinoma cell lines. J Gen Virol 80 ( Pt 7):1725-33.
25. Parkin, D. M., and F. Bray. 2006. Chapter 2: The burden of HPV-related cancers. Vaccine 24 Suppl 3:S11-25.
26. Peitsaro, P., B. Johansson, and S. Syrjanen. 2002. Integrated human papillomavirus type 16 is frequently found in cervical cancer precursors as demonstrated by a novel quantitative real-time PCR technique. J Clin Microbiol 40:886-91.
27. Reijans, M., G. Dingemans, C. H. Klaassen, J. F. Meis, J. Keijdener, B. Mulders, K. Eadie, W. van Leeuwen, A. van Belkum, A. M. Horrevorts, and G. Simons. 2008. RespiFinder: a new multiparameter test to differentially identify fifteen respiratory viruses. J Clin Microbiol 46:1232-40.
28. Ruutu, M. P., S. M. Kulmala, P. Peitsaro, and S. M. Syrjanen. 2008. The performance of the HPV16 real-time PCR integration assay. Clin Biochem.
29. Scheurer, M. E., G. Tortolero-Luna, and K. Adler-Storthz. 2005. Human papillomavirus infection: biology, epidemiology, and prevention. Int J Gynecol Cancer 15:727-46.
30. Schouten, J. P., C. J. McElgunn, R. Waaijer, D. Zwijnenburg, F. Diepvens, and G. Pals. 2002. Relative quantification of 40 nucleic acid sequences by multiplex ligation-dependent probe amplification. Nucleic Acids Res 30:e57.
31. Seedorf, K., G. Krammer, M. Durst, S. Suhai, and W. G. Rowekamp. 1985. Human papillomavirus type 16 DNA sequence. Virology 145:181-5.
32. Singh, B., A. Stoffel, S. Gogineni, A. Poluri, D. G. Pfister, A. R. Shaha, A. Pathak, G. Bosl, C. Cordon-Cardo, J. P. Shah, and P. H. Rao. 2002. Amplification of the 3q26.3 locus is associated with progression to invasive cancer and is a negative prognostic factor in head and neck squamous cell carcinomas. Am J Pathol 161:365-71.
33. Tonon, S. A., M. A. Picconi, P. D. Bos, J. B. Zinovich, J. Galuppo, L. V. Alonio, and A. R. Teyssie. 2001. Physical status of the E2 human papilloma virus 16 viral gene in cervical preneoplastic and neoplastic lesions. J Clin Virol 21:129-34.
34. Vernon, S. D., E. R. Unger, D. L. Miller, D. R. Lee, and W. C. Reeves. 1997. Association of human papillomavirus type 16 integration in the E2 gene with poor disease-free survival from cervical cancer. Int J Cancer 74:50-6.
35. Walboomers, J. M., M. V. Jacobs, M. M. Manos, F. X. Bosch, J. A. Kummer, K. V. Shah, P. J. Snijders, J. Peto, C. J. Meijer, and N. Munoz. 1999. Human papillomavirus is a necessary cause of invasive cervical cancer worldwide. J Pathol 189:12-9.
36. Wilting, S. M., P. J. Snijders, G. A. Meijer, B. Ylstra, P. R. van den Ijssel, A. M. Snijders, D. G. Albertson, J. Coffa, J. P. Schouten, M. A. van de Wiel, C. J. Meijer, and R. D. Steenbergen. 2006. Increased gene copy numbers at chromosome 20q are frequent in both squamous cell carcinomas and adenocarcinomas of the cervix. J Pathol 209:220-30.
37. Ziegert, C., N. Wentzensen, S. Vinokurova, F. Kisseljov, J. Einenkel, M. Hoeckel, and M. von Knebel Doeberitz. 2003. A comprehensive analysis of HPV integration loci in anogenital lesions combining transcript and genome-based amplification techniques. Oncogene 22:3977-84.
38. zur Hausen, H. 2002. Papillomaviruses and cancer: from basic studies to clinical application. Nat Rev Cancer 2:342-50.
39. Lee GM, Thornthwaite JT, Rasch EM: Picogram per cell determination of DNA by flow cytofluorometry, Anal Biochem 1984, 137:221-226.
40. Kulmala SM, Shabalova IP, Petrovitchev N, Syrjanen KJ, Gyllensten UB, Johansson BC, Syrjanen SM: Type-specific persistence of high-risk human papillomavirus infections in the New Independent States of the former Soviet Union Cohort Study, Cancer Epidemiol Biomarkers Prev 2007, 16:17-22.
41. Jones J, Powell NG, Tristram A, Fiander AN, Hibbitts S: Comparison of the PapilloCheck DNA micro-array Human Papillomavirus detection assay with Hybrid Capture II and PCR-enzyme immunoassay using the GP5/6+ primer set, J Clin Virol 2009, 45:100-104.

## Claims

1. A multiparameter assay to assess both the physical status of a HPV virus, more in particular HPV16 and/or HPV18, and the genomic instability of the host in a sample; said assay being **characterized in that** the multiparameter assay is performed in a single reaction; wherein the physical status of the virus is determined by typing the virus, analysis of the amount of virus present, and of the genomic integration of said virus.

2. The assay according to claim 1, wherein genomic instability of the host is determined using one or more disease progression marker genes of said host, i.e. any genomic regions/genes of said host known to be associated with an infection of the host with the virus of interest; and wherein a change in copy number of the disease progression marker genes indicates genomic instability of the host.

3. The assay according to claim 2, wherein the human disease progression marker genes are selected from any genomic regions/genes known to be associated with HPV related cancers, such the chromosome regions 3q, 5p, 20q, 17q, 8q and 1q; in particular the regions 3q and 5p. More in particular the genes within said genomic regions, such as TAF4A RNA polymerase II, TBP-associated factor, Protein kinase C binding protein 1, Nuclear receptor coactivator 6, Splicing factor (CC1.3), Tumor differentially expressed 1, and RAE1 RNA export 1 for chromosome region 20q; such as HER-2/neu (ErbB2) proto-oncogene, the metastasis-suppressor gene nm23, and the BRCA1 gene for chromosome region 17q; TERT on chromosome region 5p and TERC on chromosome region 3q; more preferably TERT on chromosome region 5p and TERC on chromosome region 3q.

4. The assay according to claim 1, wherein the viral load, typing and integration is determined by the changes in copy numbers of the HPV viral genes E6 or E7; and at least one of HPV viral genes selected from the group consisting of E1, E2, E4, E5, L1, and L2; in said sample.

5. The assay according to claim 1 wherein the viral load is determined as the ratio between the copy numbers of any one of the viral genes E6 or E7 and the copy numbers of one or more reference marker genes of the human host; in particular the average of the copy numbers of genes E6 and E7 vis-à-vis the copy numbers of the human reference marker genes β-globin and MSH2.

6. The assay according to claim 1, wherein the viral type is determined by the presence of the viral genes E6 or E7, optionally with the presence of at least one of the viral gene(s) selected from E1, E2, E4, E5, L1, and L2; or in particular by the presence of the viral genes E6 and E7, optionally with the presence of the viral gene(s) E2.

7. The assay according to claim 1 wherein the viral integration is determined by the ratio between the copy numbers of the viral genes E2 and E6; or by the ratio between the copy numbers of the viral genes E2 and E7; or as an average of the ratios between the copy numbers of the viral genes E2 and E6, and of the viral genes E2 and E7.

8. The assay according to any one of claims 2 to 7, wherein the presence of the genes is determined at the nucleic acid level, i.e. DNA or RNA; or protein level.

9. The assay according to claim 8, wherein the presence of the genes is determined at the nucleic acid level using target specific hybridizing oligonucleotide sequences i.e. a sequence specific for any one of the viral genes, disease progression marker genes or host reference marker genes as defined in claims 3, 4, 5, 6 or 7; in particular selected from the group represented in table 1.

10. The assay according to anyone of claims 4, 5 or 7, wherein the copy number is determined using multiplex ligation-dependent probes (MLPA probes) comprising said target specific hybridizing oligonucleotide sequences, in a multiplex ligation-dependent amplification assay (MLPA); said assay further optionally comprising a pre-amplification reaction.

11. The assay according to claim 10, wherein the MLPA probes include at least 1 MLPA probe specific for a viral gene selected from the group consisting of E1, E2, E4, E5, L1, and L2; at least 1 E6 MLPA probe; at least 1 TERT MLPA probe; at least one TERC MLPA probe and at least 1 MLPA probe for a human reference marker gene; or alternatively includes at least 1 MLPA probe specific for a viral gene selected from the group consisting of E1, E2, E4, E5, L1, and L2; at least 1 E7 MLPA probe; at least 1 TERT MLPA probe; at least one TERC MLPA probe and at least 1 MLPA probe for a human reference marker gene, in particular β-globin or MSH2.

12. Use of an assay according to any one of claims 1 to 11, to determine the physical status of a virus in a sample; or to monitor the disease progression of HPV related cancers, i.e. in the differentiation between regressive and progressive HPV infected lesions.

13. A kit comprising MLPA probes as defined in claim 11.

## Patentansprüche

1. Ein Multiparameter-Untersuchungsverfahren, das sich zur Beurteilung sowohl des physischen Zustands eines HPV-Virus, insbesondere von HPV16 und/oder HPV18, als auch der genomischen Instabilität des Wirtsorganismus in einer Probe eignet; wobei das besagte Multiparameter-Untersuchungsverfahren **dadurch gekennzeichnet ist, dass** es in einer einzigen Reaktion durchgeführt wird; wobei der physische Zustand des Virus durch eine Typisierung des Virus und die Analyse des zahlenmäßigen Umfangs, in welcher der Virus präsent ist, sowie der genomischen Integration des besagten Virus bestimmt wird.

2. Das Untersuchungsverfahren gemäß Anspruch 1, wobei die genomische Instabilität des Wirtsorganismus anhand von einem oder mehreren Markergenen des betreffenden Wirtsorganismus bestimmt wird, die das Fortschreiten der Krankheit anzeigen, d.h. genomische Regionen / Gene des betreffenden Wirtsorganismus, von denen bekannt ist, dass sie durch eine Infektion des Wirtsorganismus mit dem in Frage stehenden Virus betroffen sind; und wobei eine Veränderung der Vervielfältigungszahl der Markergene, die das Fortschreiten der Krankheit anzeigen, als Anzeichen für die genomische Instabilität des Wirtsorganismus gewertet wird.

3. Das Untersuchungsverfahren gemäß Anspruch 2, wobei die menschlichen Markergene, die das Fortschreiten der Krankheit anzeigen, aus genomischen Regionen / Genen auszuwählen sind, die bekanntermaßen durch HPV-bedingte Krebserkrankungen betroffen sind, wie z.B. die chromosomalen Regionen 3q, 5p, 20q, 17q, 8q und 1q; insbesondere Regionen 3q und 5p. Noch spezifischer, bestimmte Gene innerhalb der betreffenden genomischen Regionen wie TAF4A RNA Polymerase II, TBP-verbundener Faktor, Proteinkinase-C-bindendes Protein 1, nukleäre Rezeptoren Coaktivator 6, Spleißfaktor (CC1.3), Tumor differenziell exprimiert 1, und RAE1 RNA Export 1 für die chromosomale Region 20q; wie das HER-2/neu (ErbB2) Proto-Onkogen, das Metastasierungs-Suppressorgen nm23, und das BRCA1-Gen für die chromosomale Region 17q; TERT auf der chromosomalen Region 5p und TERC auf der chromosomalen Region 3q; vorzugsweise TERT auf der chromosomalen Region 5p und TERC auf der chromosomalen Region 3q.

4. Das Untersuchungsverfahren gemäß Anspruch 1, wobei die Viruslast, -typisierung und -integration anhand der Veränderungen der Vervielfältigungszahl bei den HPV-Virusgenen E6 oder E7; und wenigstens einem HPV-Virusgen aus der Gruppe E1, E2, E4, E5, L1 und L2; in der betreffenden Probe ermittelt werden.

5. Das Untersuchungsverfahren gemäß Anspruch 1, wobei die Viruslast als Verhältnis zwischen der Vervielfältigungszahl von einem der Virusgene E6 oder E7 und der Vervielfältigungszahl von einem oder mehreren der Referenzmarkergene des menschlichen Wirtsorganismus bestimmt wird; insbesondere des Durchschnitts der Vervielfältigungszahlen der Gene E6 und E7 gegenüber den Vervielfältigungszahlen der menschlichen Referenzmarkergene β-Globin und MSH2.

6. Das Untersuchungsverfahren gemäß Anspruch 1, wobei der Virustyp anhand der Präsenz der Virusgene E6 oder E7 bestimmt wird, wahlweise in Verbindung mit der Präsenz von mindestens einem der Virusgene der Gruppe E1, E2, E4, E5, L1 und L2; insbesondere anhand der Präsenz der Virusgene E6 und E7, wahlweise in Verbindung mit der Präsenz des Virusgens E2.

7. Das Untersuchungsverfahren gemäß Anspruch 1, wobei die Virusintegration durch das Verhältnis zwischen den Vervielfältigungszahlen der Virusgene E2 und E6 bestimmt wird; oder durch das Verhältnis zwischen den Vervielfältigungszahlen der Virusgene E2 und E7; oder als durchschnittliches Verhältnis der Vervielfältigungszahlen von Virusgenen E2 und E6 sowie Virusgenen E2 und E7.

8. Das Untersuchungsverfahren gemäß einem der Ansprüche 2 bis 7, wobei die Präsenz der Gene auf Nukleinsäurenebene, d.h. DNA oder RNA; oder auf Proteinebene bestimmt wird.

9. Das Untersuchungsverfahren gemäß Anspruch 8, wobei die Präsenz der Gene auf Nukleinsäurenebene mit Hilfe von zielspezifischen, hybridisierenden Oligonucleotid-Sequenzen bestimmt wird, d.h. von Sequenzen, die spezifisch jedem der betreffenden Virusgene, der das Fortschreiten der Krankheit anzeigenden Markergene oder der Referenzmarkergene des Wirtsorganismus zu entsprechen haben, welche in den Ansprüchen 3, 4, 5, 6 oder 7 definiert wurden; insbesondere denjenigen, die aus der Gruppe ausgewählt wurden, welche in Tabelle 1 dargestellt ist.

10. Das Untersuchungsverfahren gemäß einem der Ansprüche 4, 5 oder 7, wobei die Vervielfältigungszahl mit Hilfe multiplexer ligationsabhängiger Proben (MLPA-Proben), welche die besagten zielspezifischen, hybridisierenden Oligonucleotid-Sequenzen enthalten, in einem multiplexen ligationsabhängigen Amplifikationsverfahren zu ermitteln ist (MLPA); wobei das besagte Untersuchungsverfahren zusätzlich und wahlweise eine Prä-Amplifikationsreaktion umfassen kann.

11. Das Untersuchungsverfahren gemäß Anspruch 10, wobei die MLPA-Proben mindestens 1 MLPA-Probe umfassen, die spezifisch einem Virusgen aus der Gruppe E1, E2, E4, E5, L1 und L2 entspricht; mindestens 1 E6-MLPA-Probe; mindestens 1 TERT-MLPA-Probe; mindestens 1 TERC-MLPA-Probe und mindestens 1 MLPA-Probe für ein menschliches Referenzmarkergen; oder alternativ hierzu mindestens 1 MLPA-Probe, die spezifisch einem Virusgen aus der Gruppe E1, E2, E4, E5, L1 und L2 entspricht; mindestens 1 E7-MLPA-Probe; mindestens 1 TERT-MLPA-Probe; mindestens 1 TERC-MLPA-Probe und mindestens 1 MLPA-Probe für ein menschliches Referenzmarkergen, insbesondere β-Globin oder MSH2.

12. Das Untersuchungsverfahren gemäß einem der Ansprüche 1 bis 11 zur Bestimmung des physischen Zustands eines Virus in einer Probe; oder zur Beobachtung der fortschreitenden Entwicklung HPV-bedingter Krebserkrankungen, d.h. bei der Differenzierung zwischen regressiver und progressiver Entwicklung HPV-infizierter Läsionen.

13. Ein Kit mit MLPA-Proben gemäß der Definition von Anspruch 11.

## Revendications

1. Un essai à paramètres multiples pour évaluer à la fois le statut physique du virus HPV, en particulier le HPV16 et/ou le HPV18, et l'instabilité génomique de l'hôte dans un échantillon ; ledit essai étant **caractérisé par le fait que** l'essai à paramètres multiples est le résultat d'une seule réaction; alors que le statut physique du virus est déterminé en typant le virus, l'analyse de la quantité du virus présent, et l'intégration génomique dudit virus.

2. L'essai selon la revendication 1, où l'instabilité génomique de l'hôte est déterminée en utilisant un ou plusieurs gènes marqueurs de la progression de la maladie dudit hôte, c'est-à-dire toutes les régions/gènes génomiques dudit hôte connus pour être associés à une infection de l'hôte avec le virus en question ; et où le changement dans le nombre de copies des gènes marqueurs de la progression de la maladie indique l'instabilité génomique de l'hôte.

3. L'essai selon la revendication 2 où les gènes marqueurs de la progression de la maladie sont sélectionnés parmi n'importe quelle région/gène génomique connus pour être associés aux cancers HPV, comme les régions chromosomiques 3q, 5p, 20q, 17q, 8q et 1q; en particulier les régions 3q et 5p. En particulier, les gènes au sein des régions génomiques ,comme la polymérase II TAF4A RNA ,le facteur associé TBP, la protéine kinase C de la protéine de liaison 1, le coactiveur récepteur 6, le facteur d'épissage (CC1.3), la tumeur 1 exprimée différentiellement, comme le proto-oncogène HER-2/neu (ErbB2), le gène suppresseur de métastases nm23,et le gène BRCA1 pour la région chromosomique 17q; TERT sur la région chromosomique 5p et le gène suppresseur de métastases nm23, le gène BRCA1 pour la région chromosomique 17q; TERT sur la région chromosomique 5p et TERC sur la région chromosomique de préférence TERT sur la région chromosomique 5p et sur la région chromosomique 3q.

4. L'essai selon la revendication 1, où la charge virale, en la typant et où l'intégration est déterminée par les changements dans les nombres de copies des gènes viraux HPV E6 ou E7; et où au moins un des gènes viraux HPV sélectionnés dans le groupe consistant enE1, E2, E4, E5, L1, et L2; dans ledit échantillon.

5. L'essai selon la revendication 1 où la charge virale est déterminée comme le taux entre les nombres de copies des gènes viraux E6 ouE7 et les nombres de copies d'un ou plusieurs gènes marqueurs de référence de l'hôte humain; en particulier la moyenne des nombres de copies des gènes E6 et E7 vis-à-vis des nombres de copies des gènes marqueurs de référence humaine « β-globinand MSH2 ».

6. L'essai selon la revendication 1, où le type viral est déterminé par la présence de gènes viraux E6 ou E7, éventuellement avec la présence d'au moins un des gènes viraux sélectionnés dans E1, E2, E4, E5, L1, et L2 ; ou en particulier par la présence des gènes viraux E6 et E7, éventuellement avec la présence des gènes viraux E2.

7. L'essai selon les revendications où l'intégration virale est déterminée par le taux entre les nombres de copies des gènes viraux E2 et E6; ou par le taux entre les nombres de copies des gènes viraux E2 et E7; ou comme une moyenne des taux entre les nombres de copies des gènes viraux E2 et E6, et des gènes viraux E2 et E7.

8. L'essai selon les revendications 2 à 7, où la présence des gènes est déterminée par le niveau d'acide nucléique, c'est-à-dire le DNA ou RNA ; ou le niveau de protéine.

9. L'essai selon la revendication 8, où la présence des gènes est déterminée par le niveau d'acide nucléique en utilisant des séquences oligonucléotidiques hybrides spécifiques c'est-à-dire, une séquence spécifique pour un des gènes viraux, gènes marqueurs de la progression de la maladie ou les gènes marqueurs de référence de l'hôte comme il est défini dans les revendications 3, 4, 5, 6 ou 7 ; en particulier ceux qui sont sélectionnés dans le groupe représenté dans le tableau 1.

10. L'essai selon les revendications 4, 5 ou 7, où le nombre de copies utilisant les sondes multiplexes qui dépendent d'une ligature (sondes MLPA) comprenant lesdites séquences oligonuclétidiques hybrides, dans un essai d'amplification multiplex dépendant d'une ligature (MLPA) ; ledit essai comprenant en plus une réaction de préamplification.

11. L'essai selon la revendication 10, où les sondes MLPA incluent au moins 1 sonde MLPA spécifique pour un gène viral sélectionné dans un groupe consistant en E1, E2, E4, E5, L1, et L2; au moins 1 sonde MLAPA E6 ; au moins 1 sonde TERT MLPA ; au moins une sonde TERC MLPA et au moins 1 sonde MLPA pour un gène marqueur de référence humaine; ou qui inclut en alternance au moins 1 sonde MLPA spécifique pour un gène viral sélectionné dans le groupe consistant en E1, E2, E4, E5, L1, et L2; au moins une sonde MLPA 1 E7; au moins 1 sonde TERT MLPA; au moins une sonde TERC MLPA et au moins 1 sonde MLPA pour un gène marqueur de référence, en particulier « β-globinor MSH2 ».

12. L'utilisation d'un essai selon l'une des revendications 1 à 11, afin de déterminer le statut physique d'un virus dans un échantillon ; ou de contrôler la progression de la maladie des cancers HPV, c'est-à-dire la différenciation entre le HPV régressif et progressif des lésions infectées HPV.

13. Un dispositif comprenant des sondes MLPA comme il est défini dans la revendication 11.
